# FASCICULE DE BREVET EUROPEEN

(11) **EP 1 745 055 B1**
(45) Date de publication et mention de la délivrance du brevet: **11.06.2008**
(21) Numéro de dépôt: 05746986.8
(22) Date de dépôt: 15.04.2005
(51) Int. Cl.: C07F 7/12, C07F 7/18, B01J 20/22

(54) **MATERIAUX POREUX HYBRIDES ORGANIQUE-INORGANIQUES POUR LA DETECTION DE HALOGENES**
PORÖSE ORGANISCH-ANORGANISCHE HYBRIDMATERIALIEN ZUM NACHWEIS VON HALOGENEN
POROUS HYBRID ORGANIC-INORGANIC MATERIALS FOR THE DETECTION OF HALOGENS

(30) Priorité: 19.04.2004 FR 0404102
(43) Date de publication de la demande: 24.01.2007
(73) Titulaire: COMMISSARIAT A L'ENERGIE ATOMIQUE, 75015 Paris (FR); CENTRE NATIONAL DE LA RECHERCHE SCIENTIFIQUE (CNRS), 75016 Paris (FR); Université Pierre et Marie Curie (Paris VI), 75252 Paris Cédex 05 (FR)
(72) Inventeur: TRAN-THI, Thu-Hoa, F-77310 ST FARGEAU PONTHIERRY (FR); SANCHEZ, Clément, F-91440 Bures (FR); NICOLE, Lionel, F-30270 ST JEAN DU GARD (FR); HESEMANN, Peter, F-34090 MONTPELLIER (FR)
(74) Mandataire: Poulin, Gérard
(86) Numéro de dépôt international: PCT/FR2005/050248
(87) Numéro de publication internationale: WO 2005/100371

(56) Documents cités:
- EP-A2- 1 205 177
- WO-A-02/076991
- DATABASE CA [Online] CHEMICAL ABSTRACTS SERVICE, COLUMBUS, OHIO, US; IKEDA, TERUYOHI ET AL: "Dibenzoylmethane-containing organosilicon compounds and cosmetics containing them as UV absorbents" XP002306332 extrait de STN Database accession no. 128:221468 & JP 10 045768 A2 (SHIN-ETSU CHEMICAL INDUSTRY CO., LTD., JAPAN) 17 février 1998 (1998-02-17)
- DATABASE CA [Online] CHEMICAL ABSTRACTS SERVICE, COLUMBUS, OHIO, US; MOGI, HISAO ET AL: "Preparation of silyl-containing dibenzoylmethanes as UV absorbents and their intermediates" XP002306333 extrait de STN Database accession no. 120:134810 & JP 05 230071 A2 (TOSHIBA SILICONE, JAPAN) 7 septembre 1993 (1993-09-07)

## Description

### DOMAINE TECHNIQUE

La présente invention se rapporte à des composés aptes à entrer dans la constitution de matériaux poreux hybrides organique-inorganiques mésostructurés (ci-après MPHOIM) et à servir, au sein de ces matériaux, de molécules sondes pour la détection ou le dosage de composés gazeux halogénés.

Elle se rapporte également à des MPHOIM dans lesquels sont greffés ces composés par liaison covalente ou iono-covalente, à un procédé de fabrication de ces MPHOIM, ainsi qu'à des capteurs destinés à la détection ou au dosage de composés gazeux halogénés et comprenant ces MPHOIM comme matériaux sensibles.

L'invention s'applique, en particulier, à la détection et au dosage des composés gazeux halogénés utilisés dans le domaine de la micro-électronique et, plus spécialement, des complexes halogénés du bore comme, par exemple, le trifluorure de bore (BF₃) et ses dérivés (éther, dihydrate, acétonitrile, ...) et le trichlorure de bore (BCl₃).

Elle présente, donc, un intérêt tout particulier pour la surveillance et le contrôle d'une éventuelle pollution de l'atmosphère ou d'ambiances plus ou moins confinées par ce type de composés gazeux halogénés, et notamment pour la surveillance de sites industriels qui en fabriquent, qui en stockent et/ou en utilisent.

### ÉTAT DE LA TECHNIQUE ANTÉRIEURE

Depuis leur découverte par Beck et al. en 1992, les mésoporeux aux tensioactifs structurants, qui sont le plus souvent désignés par l'acronyme MTS ou plus généralement MPHOIM, suscitent l'intérêt de très nombreux laboratoires en raison de leurs structures très particulières.

Le document WO 02/076991 décrit des materiaux hybrides organiques, mésoporeux, pour séparer les gaz.

En effet, ces matériaux, qui sont obtenus en polycondensant, selon le procédé sol-gel, des réseaux d'oxydes métalliques (typiquement issus d'alcoxydes de silicium) en présence d'un agent tensioactif dont les molécules forment des micelles organisées à l'échelle du nanomètre, puis en éliminant les micelles d'agent tensioactif, ont la particularité de présenter une porosité périodiquement organisée.

Ainsi, la polycondensation des réseaux d'oxydes métalliques autour des micelles de l'agent tensioactif conduit à la formation d'une phase hybride organique-inorganique périodiquement organisée ; puis, l'élimination de la phase tensioactive libère la porosité. C'est la réplique minérale de la phase micellaire organisée qui confère au matériau une structure périodique, par exemple de type cubique, hexagonale ou lamellaire.

Cette structure peut être contrôlée, tout comme le diamètre des pores peut être ajusté au cours de la synthèse des MPHOIM. Il est notamment possible de faire varier le diamètre des pores en jouant sur la longueur des chaînes de l'agent tensioactif ou en utilisant un agent gonflant, généralement un hydrocarbure, qui va se solubiliser dans les micelles d'agent tensioactif pour en augmenter le volume.

Cette possibilité d'ajustement des pores des MPHOIM fait que ces derniers sont particulièrement intéressants pour servir de matériaux sensibles dans des capteurs destinés à détecter et quantifier des espèces moléculaires, puisqu'elle peut être mise à profit pour favoriser la pénétration et la diffusion dans le capteur des espèces moléculaires que l'on cherche à capter.

Toutefois, l'utilisation des MPHOIM comme matériaux sensibles de capteurs chimiques ou biologiques implique que soient présentes, dans les pores de ces matériaux, des molécules sondes accessibles et adaptées aux composés que l'on souhaite capter, c'est-à-dire des molécules qui sont capables d'interagir spécifiquement avec ces composés et d'en révéler la présence et, éventuellement, la concentration au sein d'un mélange complexe par l'émission d'un signal détectable.

La présente invention a, justement, pour but de fournir des composés qui sont à la fois aptes à être greffés dans un MPHOIM par liaison covalente ou iono-covalente et à servir, au sein de ce matériau, de molécules sondes pour la détection et le dosage de composés gazeux halogénés, et en particulier de complexes halogénés du bore.

Elle a, également, pour but de fournir des MPHOIM dans lesquels sont greffés ces composés par liaison covalente ou iono-covalente, ainsi que des capteurs pour la détection ou le dosage de composés gazeux halogénés et comprenant de tels MPHOIM comme matériaux sensibles.

Les demandes de brevets JP n° 10-045768, EP n° 1 205 177 et JP n° 5-230071, décrivent des β-dicétones diphénylées dont l'un des groupes phényles est substitué soit par un groupe -O(CH₂)₃-Si(OC₂H₅)₂CH₃ (JP-A-10-045768), soit par un groupe -O(CH₂)₃-Si(OC₂H₅)₃ (EP-A-1 205 177), soit encore un groupe -O(CH₂)₃-SiR_{q}(R')_{3-q} où q vaut de 0 à 3, R est un groupe alcoxy, cycloalcoxy ou aryloxy, et R' est un groupe hydrocarboné monovalent (JP-A-5-230071).

Les β-dicétones diphénylées décrites dans JP-A-10-045768 et JP-A-5-230071 sont destinées à être utilisées en tant qu'absorbeurs d'UV dans des compositions principalement cosmétiques, tandis que celle décrite dans EP-A-1 205 177 est proposée en tant que produit intermédiaire pour obtenir un conjugué utile dans des compositions dermatologiques ou cosmétiques.

En aucun cas, il n'est envisagé dans ces documents d'utiliser ces β-dicétones comme molécules sondes, en particulier dans des MPHOIM.

### EXPOSÉ DE L'INVENTION

L'invention a donc, en premier lieu, pour objet un composé répondant à au moins l'une des formules générales (I) et (II) ci-après : 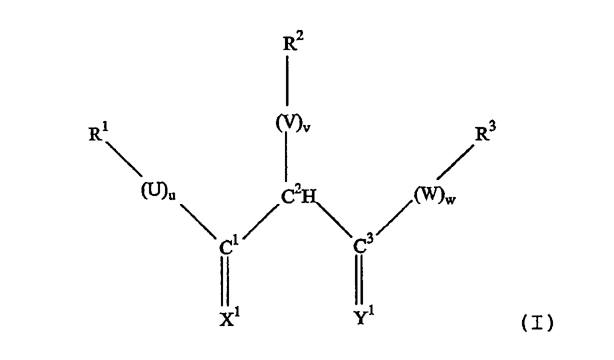 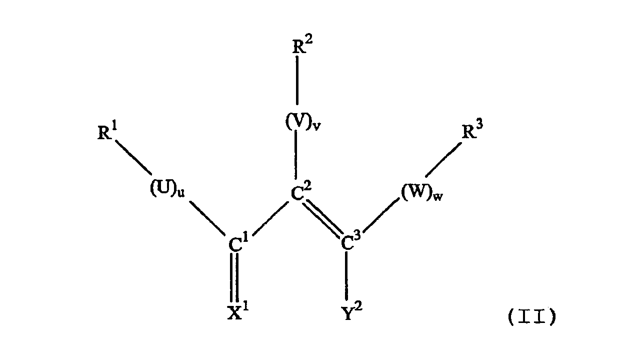 dans lesquelles :
X¹ et Y¹ représentent, indépendamment l'un de l'autre, un atome d'oxygène ou de soufre ou un groupe =NH ;
Y² représente un groupe -OH, -SH ou -NH₂ ;
u, v et w valent, indépendamment les uns des autres, 0 ou 1, à la condition toutefois qu'au moins l'un de u, v et w soit différent de 0 ;
U, V et W représentent, indépendamment les uns des autres, un groupe aryle ou hétéroaryle à un ou plusieurs cycles à 5 ou 6 chaînons chacun ;
au moins l'un de R¹, R² et R³ représente un groupe -(Z)_{z}K dans lequel :
   * z vaut 0 ou 1, Z représente un groupe divalent hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre ; et
   * K représente :
      - soit un groupe -Si(Cl)_{π.}(OR⁴)ₙ(R⁵)ₚ dans lequel :
         - m et n sont des entiers allant de 0 à 3, p est un entier allant de 0 à 2, à la condition toutefois que m + n soit égal à 1, 2 ou 3 et m + n + p soit égal à-3 ;
         - R⁴ et R⁵ représentent, indépendamment l'un de l'autre, un groupe hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre, ou bien un groupe aryle ou hétéroaryle à un cycle à 5 ou 6 chaînons ;
      - soit un groupe complexant des métaux ;
et dans lesquelles celui ou ceux de R¹, R² et R³ qui ne représentent pas un groupe -(Z)_{z}K, s'il y en a, représentent un atome d'hydrogène ou d'halogène, un groupe -CN ou -NO₂, un groupe hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre, ou bien un groupe aryle ou hétéroaryle à un cycle à 5 ou 6 chaînons ;
à l'exclusion :
- du composé de formule générale (I) dans lequel R³ représente un groupe -O(CH₂)₃-Si(OCH₂CH₃)₂CH₃ lorsque X¹ et Y¹ représentent tous deux un atome d'oxygène, u et w valent tous deux 1, U et W représentent tous deux un groupe phényle, v vaut 0 et R¹ et R² représentent tous deux un atome d'hydrogène ; et
- du composé de formule générale (I) dans lequel R³ représente un groupe -O(CH₂)₃-Si (OCH₂CH₃)₃ ou -O(CH₂)₃-Si(OCH₂CH₃)₂CH₃ lorsque X¹ et Y¹ représentent tous deux un atome d'oxygène, u et w valent 1, U et W représentent tous deux un groupe phényle, v vaut 0, R¹ représente un groupe *tert*-butyle et R² représente un atome d'hydrogène.

Ainsi, le composé selon l'invention a pour caractéristiques de comprendre :
- d'une part, un groupe fonctionnel qui est apte à réagir avec un composé gazeux halogéné et à former avec lui un complexe, qui correspond à l'ensemble X¹=C¹-C²H-C³=Y¹ dans la formule générale (I) et à l'ensemble X¹=C¹-C²=C³-Y² dans la formule générale (II) et qui est conjugué à au moins un groupe aryle ou hétéroaryle, représenté par U, V et/ou W, de manière à ce que le complexe formé soit fluorescent et donc détectable par fluorimétrie ou par spectroscopie d'absorption ; et
   ■ d'autre part, au moins un groupe fonctionnel qui permet le greffage par liaison covalente ou iono-covalente du composé dans un MPHOIM et qui correspond à la lettre K du groupe -(Z)_{z}K constitutif de l'un au moins de R¹, R² et R³.

Il est à noter que les formules générales (I) et (II) englobent des composés qui peuvent, selon le milieu dans lequel ils se trouvent, se présenter soit sous une seule forme correspondant à l'une de ces formules, soit sous deux formes tautomères en équilibre correspondant respectivement aux deux formules générales (I) et (II) dans lesquelles U, V, W, u, v, w, R¹, R², R³, X¹ sont identiques d'une formule à l'autre.

Ainsi, par exemple, les β-dicétones, c'est-à-dire les composés de formule générale (I) dans laquelle X¹ et Y¹ représentent tous les deux un atome d'oxygène, peuvent se présenter en équilibre avec leur forme énol, répondant à la formule générale (II) dans laquelle X¹ représente un atome d'oxygène et Y² représente un groupe -OH.

De manière similaire, les β-thiooxocétones, c'est-à-dire les composés de formule générale (I) dans laquelle Y¹ représente un atome d'oxygène lorsque X¹ représente un atome de soufre, et Y¹ représente un atome de soufre lorsque X¹ représente un atome d'oxygène, peuvent se présenter en équilibre de tautomérie avec leurs formes énol-énethiol répondant à la formule générale (II) dans laquelle Y² représente un groupe -OH lorsque X¹ représente un atome de soufre (énol), et Y² représente un groupe -SH lorsque X¹ représente un atome d'oxygène (énethiol).

C'est la raison pour laquelle, dans ce qui précède et ce qui suit, le composé selon l'invention est considéré comme susceptible de répondre à l'une au moins des formules générales (I) et (II).

Dans le cadre de l'invention, un groupe hydrocarboné saturé ou insaturé, linéaire ou ramifié en C₁ à C₆, peut être tout groupe alkyle, alcényle ou alcynyle comportant de 1 à 6 atomes de carbone comme, par exemple, un groupe méthyle, éthyle, propyle, isopropyle, butyle, pentyle, néopentyle, hexyle, éthylènyle, propylényle, butényle, pentényle, hexényle, méthylpentényle, buta-1,3-diényle, éthynyle, propynyle, butynyle, pentynyle ou hexynyle. De préférence, ce groupe hydrocarboné est un groupe alkyle en C₁ à C₃ ou un groupe alcényle en C₂ ou C₃.

Un groupe hydrocarboné saturé ou insaturé cyclique en C₃ à C₆ peut être tout groupe cycloalkyle ou cycloalcényle comportant de 3 à 6 atomes de carbone comme, par exemple, un groupe cyclopropyle, cyclo-butyle, cyclopentyle, cyclohexyle, cyclopropényle, cyclobutényle, cyclopentényle ou cyclohexényle. Lorsque ce groupe hydrocarboné cyclique comporte un ou plusieurs atomes d'oxygène, d'azote ou de soufre, il peut notamment être un groupe tétrahydrofuryle, tétra-hydrothiophényle, pyrrolidinyle, pipéridinyle ou dioxanyle.

Un groupe aryle ou hétéroaryle à un ou plusieurs cycles à 5 ou 6 chaînons chacun peut être tout groupe constitué d'un seul cycle comprenant 5 ou 6 atomes choisis parmi le carbone, l'oxygène, l'azote et le soufre et comportant aux moins deux doubles liaisons conjuguées, ou de plusieurs cycles de ce type accolés les uns aux autres. A titre d'exemples de groupes aryle et hétéroaryle à un seul cycle à 5 ou 6 chaînons, on peut citer les groupes cyclopentadiényle, phényle, benzyle, furyle, pyrrolyle, thiophényle, oxazolyle, isoxazolyle, thiazolyle, isothiazolyle, imidazolyle, triazolyle, pyridinyle, pyranyle, pyrazinyle et pyrimidinyle, tandis qu'à titre d'exemples de groupes aryle ou hétéroaryle à plusieurs cycles à 5 ou 6 chaînons chacun, on peut citer les groupes biphényle, phénylacétylényle, pyrényle, anthracényle, indolyle, ptéridinyle, quinolyle et isoquinolyle.

Par ailleurs, dans les formules générales (I) et (II), lorsque u vaut 0, U est absent et R¹ est lié par une liaison simple à l'atome de carbone C¹ ; lorsque v vaut 0, V est absent et R² est lié par une liaison simple à l'atome de carbone C² tandis que, lorsque w vaut 0, W est absent et R³ est lié par une liaison simple à l'atome de carbone C³.

De manière similaire, lorsque z est égal à 0, Z est absent et K est lié par une liaison simple à U, V et/ou W selon la signification R¹, R² et de R³, ou aux atomes de carbone C¹, C² et/ou C³ selon la signification de u, v et w.

Selon une disposition préférée du composé selon l'invention, celui-ci est une β-dicétone, éventuellement en équilibre avec sa forme énolique, de telles β-dicétones formant, en effet, avec les composés gazeux halogénés, et notamment avec les complexes halogénés du bore comme BF₃, des complexes dont la fluorescence est à la fois exaltée et déplacée vers de plus grandes longueurs d'onde (on parle de déplacement batochrome).

Selon une autre disposition préférée du composé selon l'invention, celui-ci répond à l'une au moins des formules générales (I) et (II) dans lesquelles :
- soit v vaut 0, tandis que u et w valent 1, auquel cas U et W représentent tous deux un groupe aryle ou hétéroaryle à un ou plusieurs cycles à 5 ou 6 chaînons chacun, et X¹, Y¹, Y², R¹, R² et R³ sont tels que précédemment définis ;
- soit u et v valent 0, tandis que w vaut 1, auquel cas seul W représente un groupe aryle ou hétéroaryle à un ou plusieurs cycles à 5 ou 6 chaînons, et X¹, Y¹ Y², R¹, R² et R³ sont tels que précédemment définis.

Conformément à l'invention, le groupe fonctionnel que comporte au minimum le composé selon l'invention pour son greffage dans un MPHOIM par liaison covalente ou iono-covalente, et qui correspond à la lettre K du groupe - (Z)_{z}K constitutif de l'un au moins de R¹, R² et R³, est choisi en fonction de l'oxyde ou des oxydes métalliques prévus pour former la phase inorganique du MPHOIM.

Dans le cadre de la présente invention, les termes "métal" et "métallique" se réfèrent aux éléments classiquement considérés comme des métaux dans la classification périodique des éléments, en particulier les éléments de transition (comme, par exemple, le titane, le zirconium, le niobium, l'yttrium, le vanadium, le chrome, le cobalt et le molybdène), les autres métaux (comme l'aluminium, le gallium, le germanium et l'étain), les lanthanides et les actinides, mais également aux métalloïdes comme le silicium, l'arsenic et le sélénium.

Ainsi, si le MPHOIM dans lequel le composé doit être greffé est prévu pour être à base d'oxyde de silicium ou d'aluminium, alors K représente un groupe -Si(Cl)ₘ(OR⁴)ₙ(R⁵)ₚ où m et n sont des entiers allant de 0 à 3, p est un entier allant de 0 à 2, m + n est égal à 1, 2 ou 3, m + n + p est égal à 3, et R⁴ et R⁵ sont tels que définis ci-avant.

Dans ce cas, K est, de préférence, choisi parmi les groupes trichlorosilyle -SiCl₃, dichloro-alcoxysilyle -SiCl₂(OR⁴), chlorodialcoxysilyle -SiCl (OR⁴)₂ et trialcoxysilyle -Si(OR⁴)₃ où R⁴ est un groupe alkyle en C₁ à C₆, de préférence en C₁ à C₃ et, mieux encore, un groupe éthyle.

En variante, si le MPHOIM dans lequel le composé doit être greffé est prévu pour être à base d'un oxyde autre que de silicium comme, par exemple, un oxyde de titane, d'aluminium de zirconium, de niobium, de vanadium, d'yttrium ou de cérium, alors K représente un groupe complexant des métaux.

Dans ce qui précède et ce qui soit, on entend par "groupe complexant des métaux", tout groupe chélatant ou polydente présentant plusieurs fonctions choisies parmi -OH, -COOH, -NH₂, =NOH, -SH, -PO₃H₂, -PO₂H, =O, =S, =N-, -NH- et -NH₂ et aptes à former une liaison dative ou de coordination avec un métal, ce groupe étant, de préférence, un groupe hydrocarboné saturé ou insaturé, linéaire ou ramifié en C₁ à C₆ ou cyclique en C₃ à C₆ substitué par une ou plusieurs des fonctions précitées.

Bien entendu, dans le cas où le composé est destiné à être greffé dans un MPHOIM dont la phase inorganique est prévue pour être constituée de deux oxydes métalliques différents, il est possible que le composé comprenne deux groupes fonctionnels différents pour son greffage par liaison covalente ou iono-covalente dans ce MPHOIM.

Ainsi, par exemple, pour un MPHOIM à base d'oxyde de silicium et d'oxyde de zirconium, le composé peut être tel qu'au moins un de R¹, R² et R³ représente un groupe -(Z)_{z}K dans lequel K est un groupe -Si(Cl)ₘ(OR⁴)ₙ(R⁵)ₚ tel que précédemment défini, et/ou qu'au moins de R¹, R² et R³ représente un groupe -(Z)_{z}K dans lequel K est un groupe complexant des métaux.

Selon une première disposit ion particulièrement préférée du composé selon l'invention, celui-ci répond à l'une au moins des formules générales (I) et (II) dans lesquelles :
X¹ et Y¹ représentent tous deux un atome d'oxygène, Y² représente un groupe -OH ;
v vaut 0, u et w valent 1 ;
U et W représentent un groupe phényle ;
R¹ représente un atome d'hydrogène ou d'halogène, un groupe -CN ou -NO₂, un groupe hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre, ou bien un groupe aryle ou hétéroaryle à un cycle à 5 ou 6 chaînons, R² représente un atome d'hydrogène, tandis que R³ représente un groupe -(Z)_{z}K dans lequel z vaut 1, Z représente un groupe divalent hydrocarboné linéaire, saturé ou insaturé, en C₁ à C₃, et K est tel que défini précédemment.

Un exemple d'un tel composé, qui est adapté à être greffé dans un MPHOIM à base de silicium, est la 1-phényl-3-[4-(2-triéthoxysilyl-vinyl)-phényl]-propane-1,3-dione.

Selon une autre disposition particulièrement préférée du composé selon l'invention, celui-ci répond à l'une au moins des formules générales (I) et (II) dans lesquelles :
X¹ et Y¹ représentent tous deux un atome d'oxygène, Y² représente un groupe -OH ;
u et v valent 0, w vaut 1 ;
W représente un groupe phényle ;
R¹ représente un atome d'hydrogène ou d'halogène, un groupe -CN ou -NO₂, un groupe hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre, ou bien un groupe aryle ou hétéroaryle à un cycle à 5 ou 6 chaînons, R² représente un atome d'hydrogène, tandis que R³ représente un groupe -(Z)_{z}K dans lequel z vaut 1, Z représente un groupe hydrocarboné divalent linéaire, saturé ou insaturé, en C₁ à C₃, et K est tel que défini précédemment.

Un exemple d'un tel composé, qui est également adapté à être greffé dans un MPHOIM à base de silicium, est la 1-[4-(2-triéthoxysilyl-vinyl)-phényl]-butane-1,3-dione.

Les composés selon l'invention peuvent être obtenus par des procédés de synthèse connus de l'état de la technique comme des réactions d'acylation de cétones (C.H. Hauser, F.W. Swamer, J.T. Adams, Org. React., 8, 1954, 59-196) ou la dérivatisation de β-dicétones (T.M. Harris, C.M. Harris, Org. React., 17, 1969, 155-211). Les β-hydroxy-cétones accessibles par condensation aldolique (A.T. Nielsen, W.J. Houlihan, Org. React., 16, 1968, 1-438) sont des produits intermédiaires importants pour la synthèse de β-dicétones. De nombreuses variantes de la condensation aldolique ont été décrites, impliquant notamment des énolates de bore (C.J. Cowden, I.Paterson, Org. React., 51, 1997, 3-200), des énolates d'étain (T. Mukaiyama, S. Kobayashi, Org. React., 46, 1994, 1-103) ou l'activation du couplage d'énoxysilanes avec des aldéhydes par des acides de Lewis comme le tétrachlorure de titane (T. Mukaiyama, Org. React., 28, 1982, 203-331).

Les β-dicétones peuvent ensuite permettre d'accéder à des composés contenant des entités complexantes azotées (S.G. McGeachin, Can. J. Chem., 46, 1968, 1903-1912 ; N. Kuhn, S. Fuchs, M. Steimann, Eur. J. Inorg. Chem., 2001, 359-361) ou soufrées (S. Lanza, F. Nicolo, F. Tresoldi, Eur. J. Inorg. Chem., 2002, 1049-1055).

L'invention a, également, pour objet un MPHOIM qui est susceptible d'être obtenu par un procédé de type sol-gel qui comprend :
a) l'hydrolyse d'au moins un composé métallique de formule générale (III) ci-après :

   M(Cl)_{q}(OR⁶)ᵣ (III)

   dans laquelle :
   M est un métal comme défini précédemment ;
   q et r sont des entiers allant de 0 à la valence de M, à la condition toutefois que q + r soit égal à cette valence ;
   R⁶ représente un groupe organique ;
   en solution dans un mélange d'eau, d'un solvant organique et d'un acide;
b) la condensation du produit obtenu à l'étape a) en solution dans un mélange comprenant, outre de l'eau, ledit solvant organique et ledit acide, un agent tensioactif ;
c) la réaction du produit obtenu à l'étape b) avec au moins un composé répondant à l'une au moins des formules générales (I) et (II) représentées ci-avant, dans lesquelles :
   X¹ et Y¹ représentent, indépendamment l'un de l'autre, un atome d'oxygène ou de soufre ou un groupe =NH ;
   Y² représente un groupe -OH, -SH ou -NH₂ ;
   u, v et w valent, indépendamment les uns des autres, 0 ou 1, à la condition toutefois qu'au moins l'un de u, v et w soit différent de 0 ;
   U, V et W représentent, indépendamment les uns des autres, un groupe aryle ou hétéroaryle à un ou plusieurs cycles à 5 ou 6 chaînons chacun ;
   au moins l'un de R¹, R² et R³ représente un groupe -(Z)_{z}K dans lequel z vaut 0 ou 1, Z représente un groupe divalent hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre ; et dans lequel :
      * lorque M est du silicium ou de l'aluminium dans le composé de formule générale (III) utilisé à l'étape a), alors K représente un groupe -Si(Cl)ₘ(OR⁴)ₙ(R⁵)ₚ dans lequel m et n sont des entiers allant de 0 à 3, p est un entier allant de 0 à 2, à la condition toutefois que m + n soit égal à 1, 2 ou 3 et m + n + p soit égal à 3 ; R⁴ et R⁵ représentent, indépendamment l'un de l'autre, un groupe hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre, ou bien un groupe aryle ou hétéroaryle à un cycle à 5 ou 6 chaînons ; tandis que
      * lorsque M est un métal autre que le silicium dans le composé de formule générale (III) utilisé à l'étape a), alors K représente un groupe complexant des métaux ;
      et dans lesquelles celui ou ceux de R¹, R² et R³ qui ne représentent pas un groupe -(Z)_{z}K, s'il y en a, représentent un atome d'hydrogène ou d'halogène, un groupe -CN ou -NO₂, un groupe hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre, ou bien un groupe aryle ou hétéroaryle à un cycle à 5 ou 6 chaînons ;
d) un traitement thermique du produit obtenu à l'étape c) ;
les étapes a), b) et c) pouvant être réalisées simultanément.

Selon une disposition préférée de l'invention, le métal M du composé de formule générale (III) utilisé à l'étape a) est choisi parmi le silicium, l'aluminium, le zirconium, le titane, le niobium, l'yttrium, le vanadium et le cérium, tandis que le groupe organique R⁶ de ce composé est un groupe alkyle en C₁ à C₆, de préférence en C₁ à C₃ et, mieux encore, en C₂.

De préférence, le métal M du composé de formule générale (III) est du silicium.

Dans ce cas, K du groupe -(Z)_{z}K constitutif de l'un au moins de R¹, R² et R³ du composé répondant à l'une au moins des formules générales (I) et (II) représente, de préférence, un groupe choisi parmi les groupes -SiCl₃, -SiCl₂(OR⁴), -SiCl(OR⁴)₂ et -Si(OR⁴)₃ où R⁴ est un groupe alkyle en C₁ à C₆, de préférence en C₁ à C₃ et, mieux encore, un groupe éthyle.

Selon un mode préféré de mise en oeuvre de ce procédé, l'étape a) est réalisée en utilisant un alcool comme solvant organique, de préférence de l'éthanol, et de l'acide chlorhydrique comme acide, et en chauffant la solution de composé de formule générale (III), par exemple à une température de l'ordre de 60 à 80°C pendant environ 1 heure.

Selon également ce mode de mise en oeuvre préféré, l'étape b) est réalisée à température ambiante en utilisant du bromure de cétyltriméthylammonium comme agent tensioactif.

Selon toujours ce mode de mise en oeuvre préféré, l'étape c) est, elle, réalisée en faisant réagir à température ambiante le composé répondant à l'une au moins des formules générales (I) et (II) en solution dans un solvant organique, avantageusement du tétrahydrofurane, avec le produit obtenu à l'étape b).

A l'étape b), il est possible d'ajouter, à la solution comprenant de l'eau, un solvant organique, un acide et un agent tensioactif, un deuxième solvant organique apte à favoriser, à l'étape c), la solubilisation du composé répondant à l'une au moins des formules (I) et (II), auquel cas ce deuxième solvant organique est le même que celui dans lequel se trouve ce composé lorsqu'il est mis à réagir en solution, à l'étape c), avec le produit obtenu à l'étape b).

Selon encore ce mode de mise en oeuvre préféré du procédé, le traitement thermique de l'étape d) consiste à soumettre le produit obtenu à l'étape c) à une température de 120 à 160°C pendant une durée de 24 à 72 heures de manière à obtenir une évaporation des différents solvants présents dans ce produit et une consolidation de sa phase inorganique.

Ce traitement thermique est avantageusement suivi d'un lavage, par exemple, par de l'éthanol en vue d'éliminer du matériau l'agent tensioactif et les autres résidus qu'il est susceptible de contenir.

De manière particulièrement préférée, le procédé qui vient d'être décrit comprend, de plus, entre les étapes c) et d), une étape dans laquelle le produit obtenu à l'étape c) est mis sous la forme d'un film mince, par exemple par la technique de dépôt par trempage ("dip coating"), par dépôt à la tournette ("spin coating") ou dépôt par pulvérisation ("spray coating") sur un support de type lame de quartz, de verre ou de silicium, en vue de son utilisation comme matériau sensible dans un capteur.

Bien entendu, le produit obtenu à l'étape c) peut tout aussi bien être mis sous la forme d'un bloc monolithique si l'usage auquel il est destiné le justifie.

Le matériau ainsi obtenu est un matériau poreux, qui est à la fois inorganique et organique, qui présente une porosité organisée à l'échelle du nanomètre conférant auxdits films ou monolithes une structure périodique de type cubique, mixte cubique-2D hexagonale, 2D hexagonale ou lamellaire, et dont les pores logent le groupe fonctionnel du composé répondant à l'une au moins des formules générales (I) et (II) qui est apte à réagir avec un composé gazeux halogéné et à former avec lui un complexe détectable par spectrométrie de fluorescence ou spectrométrie d'absorption.

La structure périodique du matériau peut avantageusement être contrôlée, notamment en jouant sur les proportions respectives du composé de formule générale (III), du tensioactif et du composé répondant à l'une au moins des formules générales (I) et (II), tout comme peut l'être le diamètre des pores de la porosité organisée par le choix de l'agent tensioactif.

Par le procédé qui vient d'être décrit, il est possible de réaliser un MPHOIM formé de plusieurs oxydes métalliques différents, auquel cas l'étape a) du procédé comprend l'hydrolyse de plusieurs composés de formule générale (III): M¹(Cl)_{q1}(OR⁶)ᵣ₁, M²(Cl)_{q2}(OR⁷)ᵣ₂, ..., Mⁿ(Cl)_{qn}(ORⁿ)ᵣₙ, dans lesquelles :
M¹, M², ..., Mⁿ représentent des métaux différents les uns des autres ;
q1 et r1 sont des entiers allant de 0 à la valence de M¹ avec q1 + r1 qui est égal à cette valence;
q2 et r2 sont des entiers allant de 0 à la valence de M² avec q2 + r2 qui est égal à cette valence ;
qn et rn sont des entiers allant de 0 à la valence de Mⁿ avec qn + rn qui est égal à cette valence ; et
R⁶, R⁷, ..., Rⁿ représentent indépendamment les uns des autres un groupe organique.

Selon la nature de M¹, M², ..., Mⁿ, l'étape c) peut être réalisée en utilisant un seul composé répondant à l'une au moins des formules générales (I) et (II), si celui-ci présente le ou les groupements fonctionnels adaptés à son greffage dans ce MPHOIM, ou un mélange de plusieurs composés répondant à l'une au moins des formules générales (I) et (II).

L'invention a, aussi, pour objet un procédé de fabrication d'un MPHOIM utile comme matériau sensible dans un capteur pour la détection ou le dosage de composés gazeux halogénés, ce procédé étant tel que précédemment défini.

L'invention a, également, pour objet, un capteur utile pour la détection ou le dosage de composés gazeux halogénés, comprenant un MPHOIM tel que précédemment défini, en tant que matériau sensible.

De préférence, ce capteur est destiné à la détection ou au dosage des complexes halogénés du bore, en particulier BF₃ et BCl₃.

L'invention a, encore, pour objet, l'utilisation d'un composé répondant à l'une au moins des formules générales (I) et (II) représentées ci-avant, dans lesquelles :
X¹ et Y¹ représentent, indépendamment l'un de l'autre, un atome d'oxygène ou de soufre ou un groupe =NH ;
Y² représente un groupe -OH, -SH ou -NH₂ ;
u, v et w valent, indépendamment les uns des autres, 0 ou 1, à la condition toutefois qu'au moins l'un de u, v et w soit différent de 0 ;
U, V et W représentent, indépendamment les uns des autres, un groupe aryle ou hétéroaryle à un ou plusieurs cycles à 5 ou 6 chaînons chacun ;
au moins l'un de R¹, R² et R³ représente un groupe -(Z)_{z}K dans lequel :
   * z vaut 0 ou 1, Z représente un groupe divalent hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre ; et
   * K représente :
      - soit un groupe -Si(Cl)ₘ(OR⁴)ₙ(R⁵)ₚ dans lequel :
         - m et n sont des entiers allant de 0 à 3, p est un entier allant de 0 à 2, à la condition toutefois que m + n soit égal à 1, 2 ou 3 et m + n + p soit égal à 3 ;
         - R⁴ et R⁵ représentent, indépendamment l'un de l'autre, un groupe hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre, ou bien un groupe aryle ou hétéroaryle à un cycle à 5 ou 6 chaînons ;
      - soit un groupe complexant des métaux;
et dans lesquelles celui ou ceux de R¹, R² et R³ qui ne représentent pas un groupe -(Z)_{z}K, s'il y en a, représentent un atome d'hydrogène ou d'halogène, un groupe -CN ou -NO₂, un groupe hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre, ou bien un groupe aryle ou hétéroaryle à un cycle à 5 ou 6 chaînons ; comme molécule sonde pour la détection ou le dosage de composés gazeux halogénés, notamment des complexes halogénés du bore, en particulier BF₃ et BCl₃.

L'invention sera mieux comprise à la lecture du complément de description qui suit, qui se rapporte à des exemples de synthèse de composés et de MPHOIM conformes à l'invention ainsi qu'à des exemples de mise en évidence de l'utilité de tels MPHOIM comme matériaux sensibles d'un capteur pour la détection de composés gazeux halogénés, et qui se réfère aux dessins annexés.

Il va de soi que ce complément de description est donné à titre d'illustration de l'invention et ne doit en aucun cas être considéré comme une limitation de l'objet de l'invention.

### BRÈVE DESCRIPTION DES DESSINS

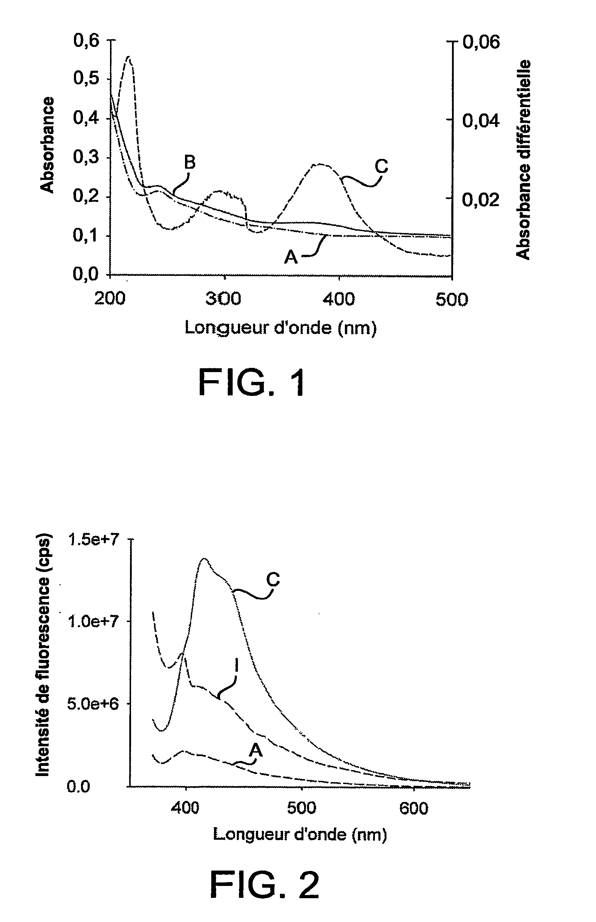

La figure 1 est un graphique illustrant l'évolution du spectre d'absorbance d'un film mince constitué par un MPHOIM conforme à l'invention, de structure cubique, tel qu'obtenu en l'absence de BF₃ (courbe A) et après réaction totale du composé présent comme molécule sonde dans ce film avec du BF₃ (courbe B), ainsi que l'absorbance différentielle de ce film (courbe C).

La figure 2 est un graphique illustrant l'évolution du spectre de fluorescence d'un film mince constitué par un MPHOIM conforme à l'invention, de structure cubique, tel qu'obtenu en l'absence de BF₃ (courbe A), après réaction partielle et après réaction totale du composé présent en tant que molécule sonde dans ce film avec du BF₃ (courbes I et C), pour une longueur d'onde d'excitation de 356 nm.

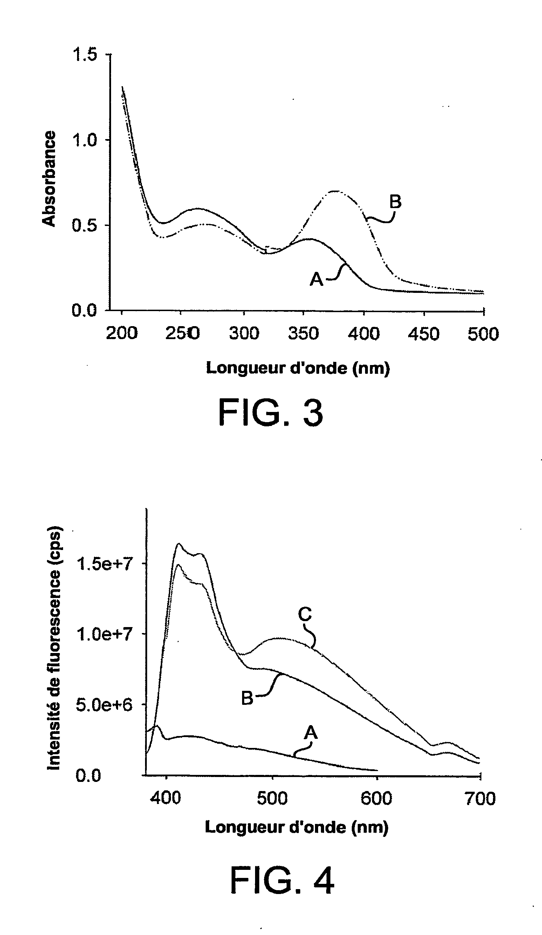

La figure 3 est un graphique illustrant l'évolution du spectre d'absorbance d'un film mince constitué par un MPHOIM conforme à l'invention, de structure lamellaire, tel qu'obtenu en l'absence de BF₃ (courbe A) et après réaction totale du composé présent en tant que molécule sonde dans ce film avec du BF₃ (courbe B).

La figure 4 est un graphique illustrant l'évolution du spectre de fluorescence d'un film mince constitué par un MPHOIM conforme à l'invention, de structure lamellaire, tel qu'obtenu en l'absence de BF₃ pour une longueur d'onde d'excitation de 356 nm (courbe A) et après réaction totale du composé présent en tant que molécule sonde dans ce film avec du BF₃, pour une longueur d'onde d'excitation respectivement de 356 nm (courbe B) et de 375 nm (courbe C).

### EXPOSÉ DÉTAILLÉ DE MODES DE RÉALISATION PARTICULIERS

### Exemple 1 : Synthèse de la 1-phényl-3-[4-(2-triéthoxysilyl-vinyl)-phényl]-propane-1,3-dione

On obtient le composé titre, ou composé 1, de formule particulière (Ia) ci-après : 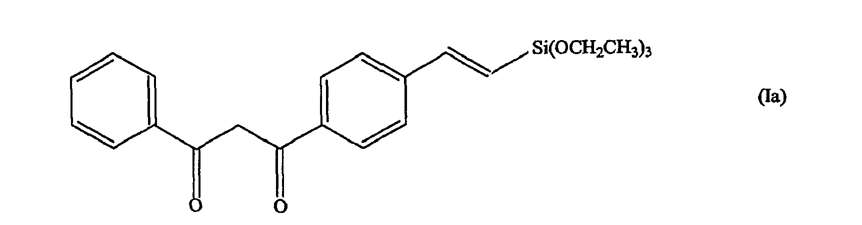 en faisant réagir la 1-phényl-3-(4-bromophënyl)-propane-1,3-dione, ou composé **5**, avec du triéthoxyvinylsilane.

### 1.1. Synthèse du composé 5

On prépare le composé **5** en faisant réagir la 4-bromoacétophénone (2,99 g, 15 mmole) avec du méthyl benzoate (4,08 g, 30 mmole) selon le protocole opératoire décrit par V.V. Popic, S.M. Korneev, V.A. Nicolaev, I.K. Korobitsyna dans Synthesis, 1991, 195-197.

On obtient ainsi 2,59 g (8,6 mmole) du composé **5**.

### 1.2. Synthèse du composé 1

Dans un bicol sous argon contenant 2,0 g (6,6 mmole) du composé **5**, 5,9 mg (0,026 mmole) d'acétate de palladium et 48,0 mg (0,16 mmole) de tri-o-toluyl-phosphine P(*o*-C₆H₄CH₃)₃, on ajoute 20 ml de diméthylformamide (DMF) fraîchement distillé, 2,8 ml (20,0 mmole) de triéthylamine et 1,7 ml (8,0 mmole) de triéthoxyvinylsilane. La solution résultante est chauffée à 100°C et laissée à cette température pendant 18 heures, après quoi on laisse le mélange réactionnel revenir à la température ambiante et on le filtre. Les solvants sont évaporés sous vide, et le résidu hétérogène obtenu est dissous dans de l'éther diéthylique. Après filtration et évaporation du solvant, on obtient 2,5 g (5,7 mmole) du composé 1, qui se présente sous la forme d'une huile orange hautement visqueuse, avec un rendement de 87%.
**RMN ¹H (CDCl₃, δ ppm)** : 1,26 (t, 9H, J=7, OHz), 3,91 (q, 6H, J=7, OHz), 6,32 (d, 1H, J=19,3Hz), 6,86 (s, 1H), 7,08 (d, 1H, J=19,4Hz), 7,47-7,59 (m, 5H), 7,95-7,99 (m, 5H), 11,69 (bs, 1H)
**RMN ¹³C (CDCl₃, δ ppm) :** 18,3, 58, 7, 93,2, 121,1, 127,0, 127,2, 127,5, 128,7, 132,5, 135,4, 135,5, 141,4, 147,7, 184, 8, 186,0
**RMN ²⁹Si (CDCl₃, δ ppm) :** -57,2 (s)
**FT-IR (KBr, Vₘₐₓ/cm⁻¹) :** 2975, 2888, 1603, 1077, 960, 821, 769
**Masse (Fab) (M+H)⁺:** calculée : 413,1821 ; trouvée : 413,1784
**Analyse élémentaire :** calculés : C=66,96%, H=6,84 ; trouvés : C=67,24%, H=6,86%

### Exemple 2 : Synthèse de la 1-[4-(2-triéthoxysilylvinyl)-phényl]-butane-1,3-dione

On obtient le composé titre, ou composé 2, de formule particulière (Ib) ci-après: 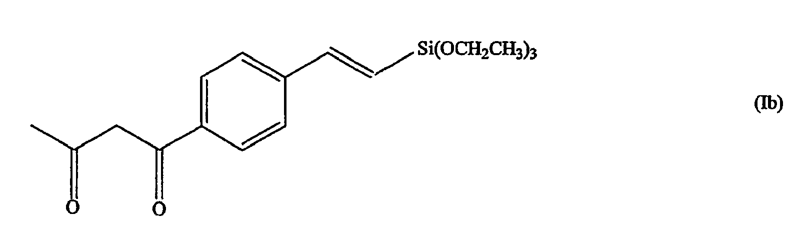 en faisant réagir la 1-[4-bromophényl]-butane-1,3-dione, ou composé **6**, avec du triéthoxyvinylsilane.

### 2.1. Synthèse du composé 6

On prépare le composé **6** en faisant réagir la 4-bromoacétophénone (4,98 g, 25 mmole) avec l'acétate d'éthyle (4.41 g, 50 mmole) selon le protocole opératoire décrit par V.V. Popic, S.M. Korneev, V.A. Nicolaev, I.K. Korobitsyna dans Synthesis, 1991, 195-197.

On obtient ainsi 4,88 g (20,3 mmole) du composé **6**.

### 2.2. Synthèse du composé 2

Dans un bicol sous argon contenant 5,0 g (20,9 mmole) du composé **6**, 14 mg (0,063 mmole) d'acétate de palladium et 75 mg (0,25 mmole) de P (*o*-C₆H₉CH₃)₃, on ajoute 40 ml de DMF fraîchement distillé, 8,7 ml (63 mmole) de triéthylamine et 5,45 ml (25,7 mmole) de triéthoxyvinylsilane. La solution résultante est chauffée à 100°C et laissée à cette température pendant 18 heures, après quoi on laisse le mélange réactionnel revenir à la température ambiante et on le filtre. Les solvants sont évaporés sous vide, et le résidu hétérogène obtenu est dissous dans de l'éther diéthylique. Après filtration et évaporation du solvant, on obtient 6,84 g (19,5 mmole) du composé 2, qui se présente sous la forme d'une huile orange hautement visqueuse, avec un rendement de 93%.
**RMN 1H (CDCl₃, δ ppm) :** 1, 25 (t, 9H, J=7,0Hz) , 2, 17 (s, 3H), 3,87 (q, 6H, J=7,0Hz), 6,15 (s, 1H), 6,27 (d, 1H, J=19,3Hz), 7,21 (d, 1H, 19,4Hz), 7,48-7,53 (m, 2H), 7,81-7,86 (m, 2H), 11,69 (bs, 1H) ;
**RMN ¹³C (CDCl₃, δ ppm) :** 18,2, 25,9, 58,7, 96,7, 121,0, 126,9, 127,3, 134,7, 141,2, 147,7, 182,3, 194,0 ;
**FT-IR (KBr, Vₘₐₓ/cm⁻¹) :** 2975, 2926, 2887, 1606, 1295, 1167, 1080, 962, 829, 783 ;
**Masse (Fab) (M+H)⁺ :** calculée : 351,1627 ; trouvée : 351,1617.

### Exemple 3 : Synthèse de MPHOIM conformes à l'invention

On réalise des MPHOIM conformes à l'invention en préparant un sol de synthèse à partir d'une solution de tétraéthoxysilane (TEOS) préalablement hydrolysé, puis en faisant réagir le sol ainsi obtenu avec des solutions concentrées du composé 1 ou 2.

Pour ce faire, on mélange du TEOS, de l'éthanol absolu, de l'acide chlorhydrique 2M/720 et de l'eau désionisée dans un rapport molaire de 1 TEOS/3 EtOH/5.10⁻⁵ HCl/1 H₂O, et on chauffe l'ensemble à 70°C pendant 1 heure. L'ordre d'addition des différents composés est libre et sans effets sur la qualité de la solution résultante.

Le sol de synthèse est préparé par addition de bromure de cétyltriméthylammonium (CTAB), d'éthanol absolu, de tétrahydrofurane (THF), d'eau désionisée et d'acide chlorhydrique 2M/36 ou 2M (au choix comme expliqué ci-après) à la solution de TEOS préhydrolysé. Là également, l'addition des différents composés est libre et sans effets sur la qualité des solutions résultantes.

Dans le cas de l'utilisation d'acide chlorhydrique 2M/36, le rapport molaire du sol de synthèse est de 0,14 CTAB/1 TEOS/6 EtOH/9 THF/5 H₂O/0,004 HCl, et dans le cas de l'utilisation d'acide chlorydrique 2M, le rapport molaire du sol de synthèse est de 0,14 CTAB/1 TEOS/6 EtOH/9 THF/5 H₂O/0,15 HCl.

Le sol présentant la teneur en HCl la plus faible est utilisé 5 jours après sa préparation jusqu'à une dizaine de jours si il est conservé à température ambiante (et plus si il est maintenu à 4°C), tandis que le sol présentant la teneur en HCl la plus élevée est utilisé le lendemain de sa préparation jusqu'à 3-4 jours (et plus si il est maintenu à 4°C).

Dans les deux cas, on ajoute au sol de synthèse des solutions concentrées (typiquement 1 M) du composé **1** ou **2** dans le THF de manière à obtenir des solutions de rapport molaire 0,14 CTAB/1 TEOS/6 EtOH/9 THF/5 H₂O/0,004-0,15 HCl/5.10⁻³-0,15 composé **1** ou **2**, que l'on agite à température ambiante pendant 15 minutes.

Pour réaliser les MPHOIM sous forme de films minces, on utilise la technique de "dip coating" sur des supports en quartz, en verre ou en silicium. Les supports sont trempés dans le sol, puis retirés à une vitesse de 2,5 mm/s.

L'humidité relative est contrôlée au cours du séchage des films. Ainsi, elle est de 40% pendant les 30 premières secondes, puis de 70% pendant 5 minutes.

Une fois réalisés, les films sont maintenus à 140°C pendant 48 heures, puis le CTAB est éliminé par des lavages à l'éthanol absolu.

Comme le montre le tableau 1 ci-après, la structure des films ainsi obtenus est cubique, mixte cubique-2D hexagonale, 2D hexagonale et lamellaire selon le pourcentage molaire du composé **1** ou **2** par rapport au silicium (c'est-à-dire le rapport entre le nombre de moles du composé **1** ou **2** présent(s) dans les films et le nombre total de moles de silicium présent dans les films, multiplié par 100).

**TABLEAU 1**

| **Structure** | Cubique | Mixte cubique-2D hexagonale | 2D hexagonale | Lamellaire |
|---|---|---|---|---|
| **% molaire composé/Si** | 0~0,7 | ~1,0 | 1, 4~2,5 | 5,3~15,0 |

### Exemple 4 : Mise en évidence de l'utilité de MPHOIM conformes à l'invention en tant que matériaux sensibles d'un capteur pour la détection de composés gazeux halogénés

Pour mettre en évidence l'utilité des MPHOIM conformes à l'invention en tant que matériaux sensibles d'un capteur pour la détection de composés gazeux halogénés, on traite des films minces de MPHOIM tels qu'obtenus à l'exemple 3 ci-avant, et comprenant le composé **1** comme molécule sonde, éventuellement en équilibre avec sa forme énol, par du BF₃ au moyen d'un dispositif identique à celui illustré sur la figure 1 de FR-A-2 840 547.

Brièvement, ce dispositif, qui a été conçu pour permettre l'incorporation d'un composé gazeux dans les pores d'un matériau poreux, se compose d'une cuve en quartz à quatre faces optiques et d'un bouchon propre à fermer hermétiquement cette cuve.

La cuve est munie d'un rodage femelle à son extrémité supérieure.

Le bouchon comporte, lui, un rodage mâle à son extrémité inférieure, lequel est apte à s'emboîter dans le rodage femelle de la cuve pour as surer la fermeture hermétique de cette dernière, et un robinet à vide à son extrémité supérieure. Ce bouchon est, de plus, muni latéralement d'un tube coudé propre à être raccordé à une rampe à vide.

Un cylindre de téflon^{®} qui est fixé au fond de la cuve permet de maintenir verticalement un film mince recouvrant l'une ou les deux faces d'un support grâce à une rainure qu'il comporte.

Après avoir placé l'un des films minces à tester dans la cuve, 500 µl d'une solution commerciale d'éthérate de trifluorure de bore (BF₃-(Et)₂O) sont déposés au fond de cette cuve. Avant de mettre le dispositif sous vide partiel, la cuve est maintenue à basse température dans de l'azote liquide ou un bain de carboglace et d'alcool pour éviter, lors de l'établissement du vide, que la solution de BF₃-(Et)₂O ne s'évapore. Après pompage et obtention du vide désiré (10⁻⁵ torr) dans la cuve, cette dernière est isolée du reste du dispositif et plongée dans un bain d'huile maintenu à une température de 50°C permettant au complexe BF₃-(Et)₂O de se dissocier et de libérer le BF₃.

La réaction entre ce dernier et le composé **1** présent dans chaque film mince est suivi e optiquement en collectant les spectres d'absorption et de fluorescence de ce film à intervalles réguliers (soit toutes les heures).

Les résultats sont illustrés sur les figures 1 à 4 qui représentent :
figure 1: l'évolution du spectre d'absorbance d'un film mince, de structure cubique et comprenant le composé **1** comme molécule sonde, tel qu'obtenu en l'absence de BF₃ (courbe A) et après réaction totale (durée de réaction = 3 heures) du composé **1** présent dans ce film avec le BF₃ (courbe B), ainsi que l'absorbance différentielle de ce film (courbe C = courbe B - courbe A) ;
figure 2 : l'évolution du spectre de fluorescence obtenus d'un film mince, de structure cubique et comprenant le composé **1** comme molécule sonde, tel qu'obtenu en l'absence de BF₃ (courbe A), après réaction partielle (durée de réaction = 1 heure) et après réaction totale (durée de réaction = 3 heures) du composé **1** présent dans ce film avec le BF₃ (courbes I et C), pour une longueur d'onde d'excitation de 356 nm ;
figure 3 : l'évolution du spectre d'absorbance d'un film mince, de structure lamellaire et comprenant le composé **1** comme molécule sonde (partiellement sous forme énol), tel qu'obtenu en l'absence de BF₃ (courbe A) et après réaction totale (durée de réaction = 8 heures) du composé **1** présent dans ce film avec le BF₃ (courbe B) ;
figure 4 : l'évolution du spectre de fluorescence d'un film mince, de structure lamellaire et comprenant le composé **1** comme molécule sonde, en l'absence de BF₃ pour une longueur d'onde d'excitation de 356 nm (courbe A), et après réaction totale (durée de réaction = 8 heures) du composé **1** présent dans ce film avec le BF₃, pour une longueur d'onde d'excitation respectivement de 356 nm (courbe B) et de 375 nm (courbe C).

## Revendications

1. Composé répondant à l'une au moins des formules générales (I) et (II) ci-après : 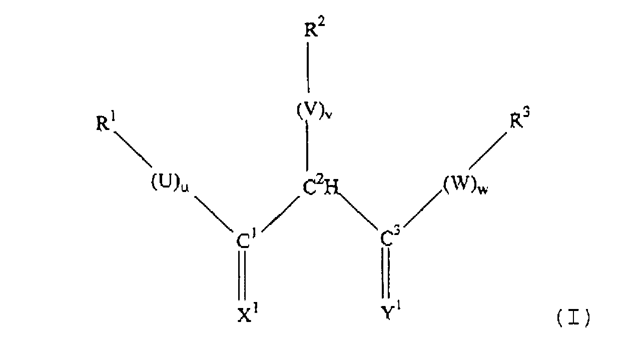 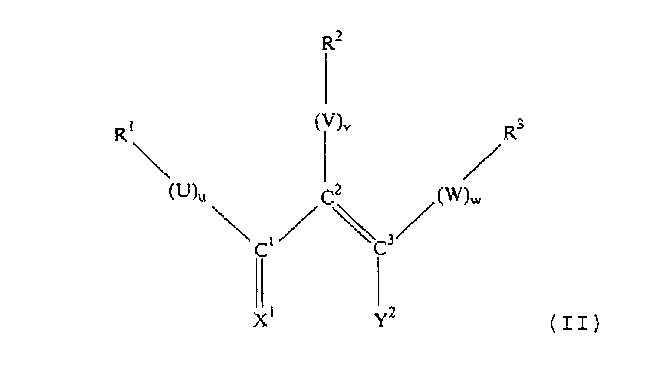 dans lesquelles :
X¹ et Y¹ représentent, indépendamment l'un de l'autre, un atome d'oxygène ou de soufre ou un groupe =NH ;
Y² représente un groupe -OH, -SH ou -NH₂ ;
u, v et w valent, indépendamment les uns des autres, 0 ou 1, à la condition toutefois qu'au moins l'un de u, v et w soit différent de 0 ;
U, V et W représentent, indépendamment les uns des autres, un groupe aryle ou hétéroaryle à un ou plusieurs cycles à 5 ou 6 chaînons chacun ;
au moins l'un de R¹, R² et R³ représente un groupe -(Z)_{z}K dans lequel:
* z vaut 0 ou 1, Z représente un groupe divalent hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre ; et
* K représente :
• soit un groupe -Si(Cl)ₘ(OR⁴)ₙ(R⁵)ₚ dans lequel :
- m et n sont des entiers allant de 0 à 3, p est un entier allant de 0 à 2, à la condition toutefois que m + n soit égal à 1, 2 ou 3 et m + n + p soit égal à 3 ;
- R⁴ et R⁵ représentent, indépendamment l'un de l'autre, un groupe hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre, ou bien un groupe aryle ou hétéroaryle à un cycle à 5 ou 6 chaînons ;
• soit un groupe complexant des métaux ; et dans lesquelles celui ou ceux de R¹, R² et R³ qui ne représentent pas un groupe -(Z)_{z}K, s'il y en a, représentent un atome d'hydrogène ou d'halogène, un groupe -CN ou -NO₂ un groupe hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre, ou bien un groupe aryle ou hétéroaryle à un cycle à 5 ou 6 chaînons ; à l'exclusion :
- du composé de formule générale (I) dans lequel R³ représente un groupe -C(CH₂)₃-Si (OCH₂CH₃)₂CH₃ lorsque X¹ et Y¹ représentent tous deux un atome d'oxygène, u et w valent tous deux 1, U et W représentent tous deux un groupe phényle, v vaut 0 et R¹ et R² représentent tous deux un atome d'hydrogène ; et
- du composé de formule générale (I) dans lequel R³ représente un groupe -O(CH₂)₃-Si(OCH₂CH₃)₃ ou -O(CH₂)₃-Si (OCH₂CH₃)₂CH₃ lorsque X¹ et Y¹ représentent tous deux un atome d'oxygène, u et w valent 1, U et W représentent tous deux un groupe phényle, v vaut 0, R¹ représente un groupe *tert-*butyle et R² représente un atome d'hydrogène.

2. Composé selon la revendication 1, qui répond à l'une au moins des formules générales (I) et (II) dans lesquelles X¹ et Y¹ représentent un atome d'oxygène, Y² représente un groupe -OH, et u, v, w, U, V, W, R¹, R² et R³ sont tels que définis précédemment.

3. Composé selon la revendication 1 ou la revendication 2, qui répond à l'une au moins des formules générales (I) et (II) dans lesquelles v vaut 0, u et w valent 1, et U, W, X², Y¹, Y², R¹, R² et R³ sont tels que définis précédemment.

4. Composé selon la revendication 1 ou la revendication 2, qui répond à l'une au moins des formules générales (I) et (II) dans lesquelles u et v valent 0, w vaut 1, et w, X¹, Y¹, Y², R¹, R² et R³ sont tels que définis précédemment.

5. Composé selon l'une quelconque des revendications 1 à 4, dans lequel K du groupe -(Z)_{z}K constitutif de l'un au moins de R¹, R² et R³ représente un groupe choisi parmi les groupes -SiCl₃, -SiCl₂(OR⁴), -SiCl(OR⁴)₂ et -Si (OR⁴)₃ où R⁴ est un groupe alkyle en C₂ à C₆, de préférence en C₁ à C₃ et, mieux encore, en C₂.

6. Composé selon l'une quelconque des revendications 1 à 4, dans lequel K du groupe - (Z)_{z}K constitutif de l'un au moins de R¹, R² et R³ représente un groupe hydrocarboné saturé ou insaturé, linéaire ou ramifié en C₁ à C₆ ou cyclique en C₃ à C₆ substitué par plusieurs fonctions choisies parmi -OH, -COOH, -NH₂, =NOH, -SH, -PO₃H₂, -PO₂H, =O, =S, =N-, -NH- et -NH₂.

7. Composé selon l'une quelconque des revendications 1 à 6, qui répond à l'une au moins des formules générales (I) et (II) dans lesquelles :
X¹ et Y¹ représentent tous deux un atome d'oxygène, Y² représente un groupe -OH ;
v vaut 0, u et w valent 1 ;
U et W représentent un groupe phényle ;
R¹ représente un atome d'hydrogène ou d'halogène, un groupe -CN ou -NO₂, un groupe hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre, ou bien un groupe aryle ou hétéroaryle à un cycle à 5 ou 6 chaînons, R² représente un atome d'hydrogène, tandis que R³ représente un groupe -(Z)_{z}K dans lequel z vaut 1, Z représente un groupe divalent hydrocarboné linéaire, saturé ou insaturé, en C₁ à C₃, et K est tel que défini précédemment.

8. Composé selon la revendication 7, qui est la 1-phényl-3-[4-(2-triéthoxysilyl-vinyl)-phényl]-propane-1,3-dione.

9. Composé selon l'une quelconque des revendications 1 à 6, qui répond à l'une au moins des formules générales (I) et (II) dans lesquelles :
X¹ et Y¹ représentent tous deux un atome d'oxygène, Y² représente un groupe -OH;
u et v valent 0, w vaut 1 ;
W représente un groupe phényle ;
R¹ représente un atome d'hydrogène ou d'halogène, un groupe -CN ou -NO₂, un groupe hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre, ou bien un groupe aryle ou hétéroaryle à un cycle à 5 ou 6 chaînons, R² représente un atome d'hydrogène, tandis que R³ représente un groupe -(Z)_{z}K dans lequel z vaut 1, Z représente un groupe hydrocarboné divalent linéaire, saturé ou insaturé, en C₁ à C₃, et K est tel que défini précédemment.

10. Composé selon la revendication 9, qui est la 1-[4-(2-triéthoxysilyl-vinyl)-phényl]-butane-1,3-dione.

11. Matériau poreux hybride organique-inorganique mésostructuré susceptible d'être obtenu par un procédé de type sol-gel qui comprend :
a) l'hydrolyse d'au moins un composé métallique de formule générale (III) ci-après :
M(Cl)_{q}(OR⁶)ᵣ (III)
dans laquelle :
M est un métal ;
q et r sont des entiers allant de 0 à la valence de M, à la condition toutefois que q + r soit égal à cette valence ; et
R⁶ représente un groupe organique ;
en solution dans un mélange d'eau, d'un solvant organique et d'un acide ;
b) la condensation du produit obtenu à l'étape a) en solution dans un mélange contenant, outre de l'eau, ledit solvant organique et ledit acide, un agent tensioactif ;
c) la réaction du produit obtenu à l'étape b) avec au moins un composé répondant à l'une au moins des formules générales (I) et (II) ci-après : 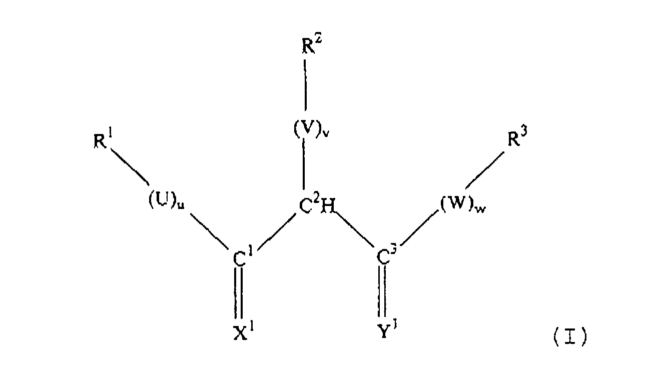 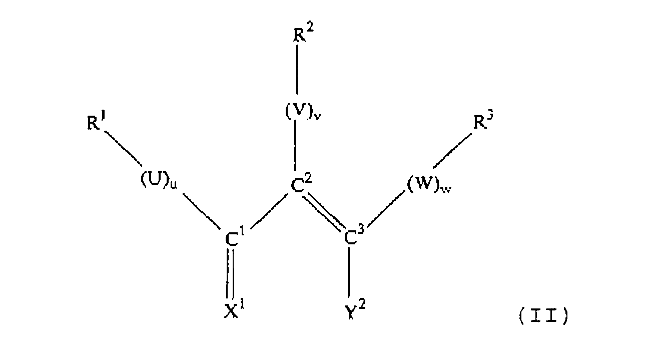 dans lesquelles :
X¹ et Y¹ représentent, indépendamment l'un de l'autre, un atome d'oxygène ou de soufre ou un groupe =NH ;
Y² représente un groupe -OH, -SE ou -NH₂ ;
u, v et w valent, indépendamment les uns des autres, 0 ou 1, à la condition toutefois qu'au moins l'un de u, v et w soit différent de 0 ;
U, V et W représentent, indépendamment les uns des autres, un groupe aryle ou hétéroaryle à un ou plusieurs cycles à 5 ou 6 chaînons chacun ;
au moins l'un de R¹, R² et R³ représente un groupe -(Z)_{z}K dans lequel z vaut 0 ou 1, Z représente un groupe divalent hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre ; et dans lequel :
* lorque M est du silicium ou de l'aluminium dans le composé de formule générale (III) utilisé à l'étape a), alors K représente un groupe -Si(Cl)ₘ(OR⁴)ₙ(R⁵)ₚ dans lequel m et n sont des entiers allant de 0 à 3, p est un entier allant de 0 à 2, à la condition toutefois que m + n soit égal à 1, 2 ou 3 et m + n + p soit égal à 3; R⁴ et R⁵ représentent, indépendamment l'un de l'autre, un groupe hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre, ou bien un groupe aryle ou hétéroaryle à un cycle à 5 ou 6 chaînons ; tandis que
* lorsque M est un métal autre que le silicium dans le composé de formule générale (III) utilisé à l'étape a), alors K représente un groupe complexant des métaux ;
et dans lesquelles celui ou ceux de R¹, R² et R³ qui ne représentent pas un groupe -(Z)_{z}K, s'il y en a, représentent un atome d'hydrogène ou d'halogène, un groupe -CN ou -NO₂, un groupe hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre, ou bien un groupe aryle ou hétéroaryle à un cycle à 5 ou 6 chaînons ;
d) un traitement thermique du produit obtenu à l'étape c) ;
les étapes a), b) et c) pouvant être réalisées simultanément.

12. Matériau poreux hybride organique-inorganique mésostructuré selon la revendication 11, qui est susceptible d'être obtenu par un procédé dans lequel le métal M du composé de formule générale (III) est choisi parmi le silicium, l'aluminium, le zirconium, le titane, le niobium, l'yttrium, le vanadium et le cérium.

13. Matériau poreux hybride organique-inorganique mésostructuré selon la revendication 11 ou la revendication 12, qui est susceptible d'être obtenu par un procédé dans lequel le groupe organique R⁶ du composé de formule générale (III) est un groupe alkyle en C₁ à C₆, de préférence en C₁ à C₃ et, mieux encore, en C₂.

14. Matériau poreux hybride organique-inorganique mésostructuré selon l'une quelconque des revendications 11 à 13, qui est susceptible d'être obtenu par un procédé dans lequel le métal M du composé de formule générale (III) est du silicium.

15. Matériau poreux hybride organique-inorganique mésostructuré selon la revendication 14, qui est susceptible d'être obtenu par un procédé dans lequel K du groupe -(Z)_{z}K constitutif de l'un au moins de R¹, R² et R³ du composé répondant à l'une au moins des formules générales (I) et (II) représente un groupe choisi parmi les groupes -SiCl₃, -SiCl₂(OR⁴), -SiCl(OR⁴)₂ et -Si(OR⁴)₃ où R⁴ est un groupe alkyle en C₁ à C₆, de préférence en C₁ à C₃ et, mieux encore, en C₂.

16. Matériau poreux hybride organique-inorganique mésostructuré selon l'une quelconque des revendications 11 à 15, qui est susceptible d'être obtenu par un procédé dans lequel l'étape a) est réalisée en utilisant un alcool comme solvant organique, de préférence de l'éthanol, et de l'acide chlorhydrique comme acide, et en chauffant la solution de composé de formule générale (III).

17. Matériau poreux hybride organique-inorganique mésostructuré selon l'une quelconque des revendications 11 à 16, qui est susceptible d'être obtenu par un procédé dans lequel l'étape b) est réalisée à température ambiante en utilisant le bromure de cétyltriméthylammonium comme agent tensioactif.

18. Matériau poreux hybride organique-inorganique mésostructuré selon l'une quelconque des revendications 11 à 17, qui est susceptible d'être obtenu par un procédé dans lequel l'étape c) est réalisée en faisant réagir à température ambiante le composé répondant à au moins l'une des formules générales (I) et (II) en solution dans un solvant organique, avec le produit obtenu à l'étape b).

19. Matériau poreux hybride organique-inorganique mésostructuré selon l'une quelconque des revendications 11 à 18, qui est susceptible d'être obtenu par un procédé dans lequel, à l'étape d), le traitement thermique consiste à soumettre le produit résultant de l'étape c) à une température de 120 à 160°C pendant une durée de 24 à 72 heures.

20. Matériau poreux hybride organique-inorganique mésostructuré selon l'une quelconque des revendications 11 à 19, qui est susceptible d'être obtenu par un procédé qui comprend, de plus, entre les étapes c) et d), une étape dans laquelle le produit obtenu à l'étape c) est mis sous la forme d'un film mince.

21. Matériau poreux hybride organique-inorganique mésostructuré selon l'une quelconque des revendications 11 à 20, qui est susceptible d'être obtenu par un procédé dans lequel l'étape a) comprend l'hydrolyse de plusieurs composés de formule générale (III) : M¹(Cl)_{q1}(OR⁶)_{r1,} M²(Cl)_{q2}(OR⁷)ᵣ₂, ..., Mⁿ(Cl)_{qn}(ORⁿ)ₘ, dans lesquelles :
M¹, M², ..., Mⁿ représentent des métaux différents les uns des autres ;
q1 et r1 sont des entiers allant de 0 à la valence de M¹ avec q1 + r1 qui est égal à cette valence ;
q2 et r2 sont des entiers allant de 0 à la valence de M² avec q2 + r2 qui est égal à cette valence ;
qn et rn sont des entiers allant de 0 à la valence de Mⁿ avec qn + rn qui est égal à cette valence ; et
R⁶, R⁷, ..., Rⁿ représentent indépendamment les uns des autres un groupe organique.

22. Procédé de fabrication d'un matériau poreux hybride organique-inorganique mésostructuré utile comme matériau sensible dans un capteur pour la détection ou le dosage de composés gazeux halogénés, qui est tel que défini dans l'une quelconque des revendications 11 à 21.

23. Capteur utile pour la détection ou le dosage de composés gazeux halogénés, comprenant un matériau poreux hybride organique-inorganique mésostructuré tel que défini dans l'une quelconque des revendications 11 à 21 en tant que matériau sensible.

24. Capteur selon la revendication 23, pour la détection ou au dosage des complexes halogénés du bore, en particulier BF₃ et BCl₃.

25. Utilisation d'un composé répondant à l'une au moins des formules générales (I) et (II) ci-après: 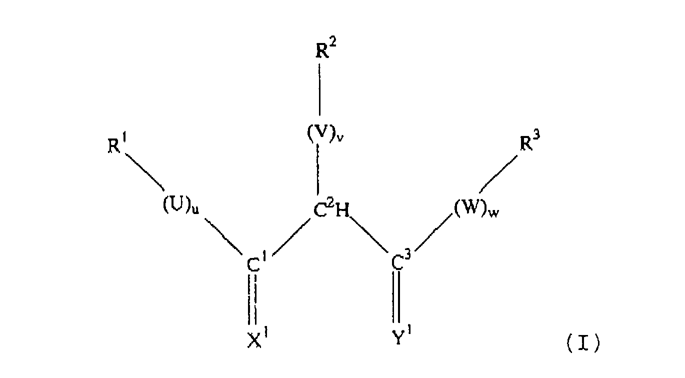 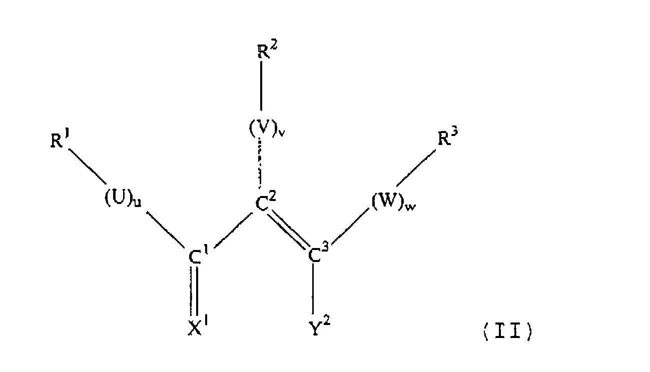 dans lesquelles :
X¹ et Y¹ représentent, indépendamment l'un de l'autre, un atome d'oxygène ou de soufre ou un groupe =NH ;
Y² représente un groupe -OH, -SH ou -NH₂ ;
u, v et w valent, indépendamment les uns des autres, 0 ou 1, à la condition toutefois qu'au moins l'un de u, v et w soit différent de 0 ;
U, V et W représentent, indépendamment les uns des autres, un groupe aryle ou hétéroaryle à un ou plusieurs cycles à 5 ou 6 chaînons chacun ;
au moins l'un de R¹, R² et R³ représente un groupe -(Z)_{z}K dans lequel:
* z vaut 0 ou 1, Z représente un groupe divalent hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre ; et
* K représente :
• soit un groupe -Si(Cl)ₘ(OR⁶)ₙ(R⁵)ₚ dans lequel :
- m et n sont des entiers allant de 0 à 3, p est un entier allant de 0 à 2, à la condition toutefois que n + n soit égal à 1, 2 ou 3 et m + n + p soit égal à 3 ;
- R⁴ et R⁵ représentent, indépendamment l'un de l'autre, un groupe hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre, ou bien un groupe aryle ou hétéroaryle à un cycle à 5 ou 6 chaînons ;
• soit un groupe complexant des métaux ; et dans lesquelles celui ou ceux de R¹, R² et R³ qui ne représentent pas un groupe -(Z)_{z}K, s'il y en a, représentent un atome d'hydrogène ou d'halogène, un groupe -CN ou -NO₂, un groupe hydrocarboné saturé ou insaturé, linéaire ou branché en C₁ à C₆, ou cyclique en C₃ à C₆, ce groupe hydrocarboné comportant éventuellement un ou plusieurs atomes d'oxygène, d'azote ou de soufre, ou bien un groupe aryle ou hétéroaryle à un cycle à 5 ou 6 chaînons ; comme molécule sonde pour la détection ou le dosage de composés gazeux halogénés.

26. Utilisation selon la revendication 25, pour la détection cu le dosage des complexes halogénés du bore, en particulier BF₃ et BCl₃.

## Claims

1. Compound corresponding to at least one of the general formulae (I) and (II) below: 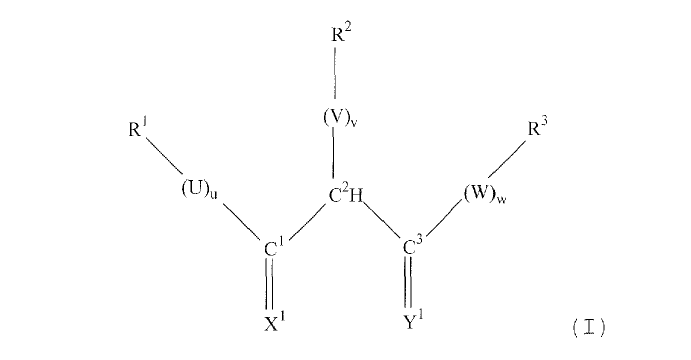 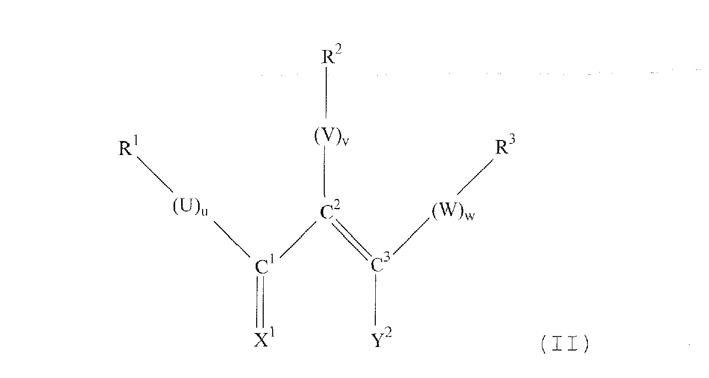 in which:
X¹ and Y¹ represent, independently of one another, an oxygen or sulphur atom or an =NH group;
Y² represents an -OH, -SH or -NH₂ group;
u, v and w are, independently of one another, 0 or 1, with the proviso, however, that at least one of u, v and w is other than 0;
U, V and W represent, independently of one another, an aryl or heteroaryl group having one or more rings, each having 5 or 6 ring members;
at least one of R¹, R² and R³ represents a group -(Z)_{z}K in which:
* z is 0 or 1, Z represents a C₃ to C₆ cyclic, or C₁ to C₆ linear or branched, saturated or unsaturated divalent hydrocarbon group, this hydrocarbon group optionally containing one or more oxygen, nitrogen or sulphur atoms; and
* K represents:
• either a group -Si(Cl)ₘ(OR⁴)ₙ(R⁵)ₚ in which:
- m and n are integers ranging from 0 to 3, p is an integer ranging from 0 to 2, with the proviso, however, that m + n is equal to 1, 2 or 3 and m + n + p is equal to 3;
- R⁴ and R⁵ represent, independently of one another, a C₃ to C₆ cyclic, or C₁ to C₆ linear or branched, saturated or unsaturated hydrocarbon group, this hydrocarbon group optionally containing one or more oxygen, nitrogen or sulphur atoms, or else an aryl or heteroaryl group having a 5- or 6-membered ring;
• or a metal-complexing group;
and in which that or those of R¹, R² and R³ which does not or do not represent a group -(Z)_{z}K, if there is one, represent(s) a hydrogen or halogen atom, a -CN or -NO₂ group, a C₃ to C₆ cyclic, or C₁ to C₆ linear or branched, saturated or unsaturated hydrocarbon group, this hydrocarbon group optionally containing one or more oxygen, nitrogen or sulphur atoms, or else an aryl or heteroaryl group having a 5- or 6-membered ring; but excepting:
the compound of general formula (I), in which R³ represents a -O(CH₂)₃-Si(OCH₂CH₃)₂ group, when X¹ and Y¹ both represent an oxygen atom, u and w are both equal to 1, U and W both represent a phenyl group, v is equal to 0 and R¹ and R² both represent a hydrogen atom; and the compound of general formula (1) in which R³ represents a -O (CH₂)₃-Si (OCH₂CH₃) or a -O (CH₂)₃-Si (OCH₂CH₃)₂CH₃ group, when X¹ and Y¹ both represent an oxygen atom, u and w are equal to 1, U and W both represent a phenyl group, v is equal to 0, R¹ represents a *tert*-butyl group and R² represents a hydrogen atom.

2. Compound according to Claim 1, which corresponds to at least one of general formulae (I) and (II) in which X¹ and Y¹ represent an oxygen atom, Y² represents an -OH group, and u, v, w, U, V, W, R¹, R² and R³ are as defined above.

3. Compound according to Claim 1 or Claim 2, which corresponds to at least one of general formulae (I) and (II) in which v is 0, u and w are 1, and U, W, X¹, Y¹, Y², R¹, R² and R³ are as defined above.

4. Compound according to Claim 1 or Claim 2, which corresponds to at least one of general formulae (I) and (II) in which u and v are 0, w is 1, and W, X¹, Y¹, Y², R¹, R² and R³ are as defined above.

5. Compound according to any one of Claims 1 to 4, in which K of the group -(Z)_{z}K constituting at least one of R¹, R² and R³ represents a group chosen from the groups -SiCl₃, -SiCl₂(OR⁴),
- SiCl (OR⁴)₂ and -Si (OR⁴)₃ where R⁴ is a C₁ to C₆, preferably C₁ to C₃, and better still C₂, alkyl group.

6. Compound according to any one of Claims 1 to 4, in which K of the group -(Z)_{z}K constituting at least one of R¹, R² and R³ represents a C₃ to C₆ cyclic, or C₁ to C₆ linear or branched, saturated or unsaturated hydrocarbon group substituted with several functions chosen from -OH, -COOH, -NH₂, =NOH, -SH, -PO₃H₂, -PO₂H, =O, =S, =N-, -NH- and -NH₂.

7. Compound according to any one of Claims 1 to 6, which corresponds to at least one of general formulae (I) and (II) in which:
X¹ and Y¹ both represent an oxygen atom, Y² represents an -OH group;
v is 0 and u and w are 1;
U and W represent a phenyl group;
R¹ represents a hydrogen or halogen atom, a -CN or -NO₂ group, a C₃ to C₆ cyclic, or C₁ to C₆ linear or branched, saturated or unsaturated hydrocarbon group, this hydrocarbon group optionally containing one or more oxygen, nitrogen or sulphur atoms, or else an aryl or heteroaryl group having a 5- or 6-membered ring, R² represents a hydrogen atom, while R³ represents a group -(Z)_{z}K in which z is 1, Z represents a C₁ to C₃ linear, saturated or unsaturated divalent hydrocarbon group, and K is as defined above.

8. Compound according to Claim 7, which is 1-phenyl-3-[4-(2-triethoxysilylvinyl)phenyl]propane-1,3-dione.

9. Compound according to any one of Claims 1 to 6, which corresponds to at least one of general formulae (I) and (II) in which:
X¹ and Y¹ both represent an oxygen atom, Y² represents an -OH group;
u and v are 0, w is 1;
W represents a phenyl group;
R¹ represents a hydrogen or halogen atom, a -CN or -NO₂ group, a C₃ to C₆ cyclic, or C₁ to C₆ linear or branched, saturated or unsaturated hydrocarbon group, this hydrocarbon group optionally containing one or more oxygen, nitrogen or sulphur atoms, or else an aryl or heteroaryl group having a 5- or 6-membered ring, R² represents a hydrogen atom, while R³ represents a group -(Z)_{z}K in which z is 1, Z represents a C₁ to C₃ linear, saturated or unsaturated divalent hydrocarbon group, and K is as defined above.

10. Compound according to Claim 9, which is 1-[4-(2-triethoxysilylvinyl)phenyl] butane-1, 3-dione.

11. Mesostructured porous hybrid organic-inorganic material which can be obtained by means of a sol-gel type process which comprises:
a) hydrolysis of at least one metal compound of general formula (III) below :
M(Cl)_{q}(OR⁶)ᵣ (III)
in which:
M is a metal;
q and r are integers ranging from 0 to the valency of M, with the proviso, however, that q + r is equal to this valency; and
R⁶ represents an organic group;
in solution in a mixture of water, of an organic solvent and of an acid;
b) condensation of the product obtained in step a) in solution in a mixture containing, in addition to water, said organic solvent and said acid, a surfactant;
c) reaction of the product obtained in step b) with at least one compound corresponding to at least one of general formulae (I) and (II) below: 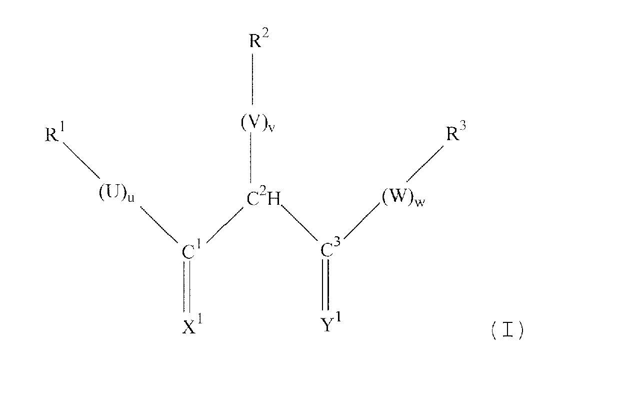 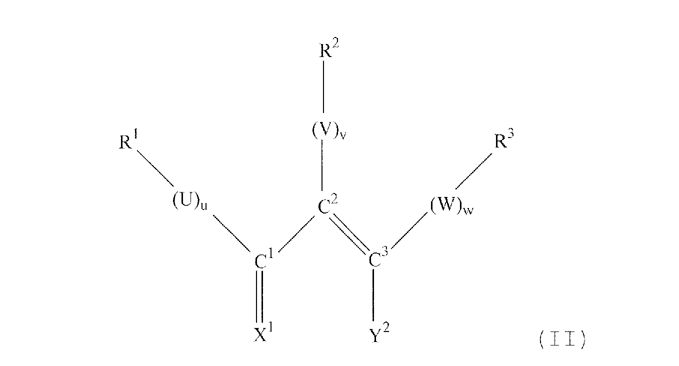 in which:
X¹ and Y¹ represent, independently of one another, an oxygen or sulphur atom or an =NH group;
Y² represents an -OH, -SH or -NH₂ group; u, v and w are, independently of one another, 0 or 1, with the proviso, however, that at least one of u, v and w is other than 0;
U, V and W represent, independently of one another, an aryl or heteroaryl group having one or more rings, each having 5 or 6 ring members;
at least one of R¹, R² and R³ represents a group -(Z)_{z}K in which z is 0 or 1, Z represents a C₃ to C₆ cyclic, or C₁ to C₆ linear or branched, saturated or unsaturated divalent hydrocarbon group, this hydrocarbon group optionally containing one or more oxygen, nitrogen or sulphur atoms; and in which:
* when M is silicon or aluminium in the compound of general formula (III) used in step a), then K represents a group -Si(Cl)ₘ(OR⁴)ₙ(R⁵)ₚ in which m and n are integers ranging from 0 to 3, p is an integer ranging from 0 to 2, with the proviso, however, that m + n is equal to 1, 2 or 3 and m + n + p is equal to 3; R⁴ and R⁵ represent, independently of one another, a C₃ to C₆ cyclic, or C₂ to C₆ linear or branched, saturated or unsaturated hydrocarbon group, this hydrocarbon group optionally containing one or more oxygen, nitrogen or sulphur atoms, or else an aryl or heteroaryl group having a 5- or 6-membered ring; whereas
* when M is a metal other than silicon in the compound of general formula (III) used in step a), then K represents a metal-complexing group; and in which that or those of R¹, R² and R³ which does not or do not represent a group -(Z)_{z}K, if there is one, represent(s) a hydrogen or halogen atom, a -CN or -NO₂ group, a C₃ to C₆ cyclic, or C₁ to C₆ linear or branched, saturated or unsaturated hydrocarbon group, this hydrocarbon group optionally containing one or more oxygen, nitrogen or sulphur atoms, or else an aryl or heteroaryl group having a 5- or 6-membered ring;
d) thermal treatment of the product obtained in step c);
it being possible for steps a), b) and c) to be carried out simultaneously.

12. Mesostructured porous hybrid organic-inorganic material according to Claim 11, which can be obtained by means of a process in which the metal M of the compound of general formula (III) is chosen from silicon, aluminium, zirconium, titanium, niobium, yttrium, vanadium and cerium.

13. Mesostructured porous hybrid organic-inorganic material according to Claim 11 or Claim 12, which can be obtained by means of a process in which the organic group R⁶ of the compound of general formula (III) is a C₁ to C₆, preferably C₁ to C₃, and better still C₂, alkyl group.

14. Mesostructured porous hybrid organic-inorganic material according to any one of Claims 11 to 13, which can be obtained by means of a process in which the metal M of the compound of general formula (III) is silicon.

15. Mesostructured porous hybrid organic-inorganic material according to Claim 14, which can be obtained by means of a process in which K of the group - (Z)_{z}K constituting at least one of R¹, R² and R³ of the compound corresponding to at least one of general formulae (I) and (II) represents a group chosen from the groups -SiCl₃, -SiCl₂(OR⁴), -SiCl (OR⁴)₂ and -Si(OR⁴)₃ where R⁴ is a C₁ to C₆, preferably C₁ to C₃, and better still C₂, alkyl group.

16. Mesostructured porous hybrid organic-inorganic material according to any one of Claims 11 to 15, which can be obtained by means of a process in which step a) is carried out using an alcohol as organic solvent, preferably ethanol, and hydrochloric acid as acid, and by hearing the solution of compound of general formula (III).

17. Mesostructured porous hybrid organic-inorganic material according to any one of Claims 11 to 16, which can be obtained by means of a process in which step b) is carried out at ambient temperature using cetyltrimethylammonium bromide as surfactant.

18. Mesostructured porous hybrid organic-inorganic material according to any one of Claims 11 to 17, which can be obtained by means of a process in which step c) is carried out by reacting, at ambient temperature, the compound corresponding to at least one of general formulae (I) and (II) in solution in an organic solvent, with the product obtained in step b).

19. Mesostructured porous hybrid organic-inorganic material according to any one of Claims 11 to 18, which can be obtained by means of a process in which, in step d), the thermal treatment consists in subjecting the product resulting from step c) to a temperature of 12.0 to 160°C for a period of 24 to 72 hours.

20. Mesostructured porous hybrid organic-inorganic material according to any one of Claims 11 to 19, which can be obtained by means of a process which comprises, in addition, between steps c) and d), a step in which the product obtained in step c) is made into the form of a thin film.

21. Mesostructured porous hybrid organic-inorganic material according to any one of Claims 11 to 20, which can be obtained by means of a process in which step a) comprises the hydrolysis of several compounds of general formula (III): M¹(Cl)_{q1}(OR⁶)ᵣ₁, M²(Cl)_{q2}(OR⁷)ᵣ₂, ..., Mⁿ(Cl)_{qn}(ORⁿ)ᵣₙ, in which:
M¹, M², ..., Mⁿ represent metals different from one another;
q1 and r1 are integers ranging from 0 to the valency of M¹ with q1 + r1 which is equal to this valency;
q2 and r2 are integers ranging from 0 to the valency of M² with q2 + r2 which is equal to this valency;
qn and rn are integers ranging from 0 to the valency of Mⁿ with qn + rn which is equal to this valency; and
R⁶, R⁷, ..., Rⁿ represent, independently of one another, an organic group.

22. Process for manufacturing a mesostructured porous hybrid organic-inorganic material which can be used as sensitive material in a sensor for the detection or quantitative determination of halogenated gaseous compounds, which is as defined in any one of Claims 11 to 21.

23. Sensor which can be used for the detection or quantitative determination of halogenated gaseous compounds, comprising a mesostructured porous hybrid organic-inorganic material as defined in any one of Claims 11 to 21 as sensitive material.

24. Sensor according to Claim 23, for the detection or quantitative determination of halogenated boron complexes, in particular BF₃ and BCl₃.

25. Use of a compound corresponding to at least one of general formulae (I) and (II) below: 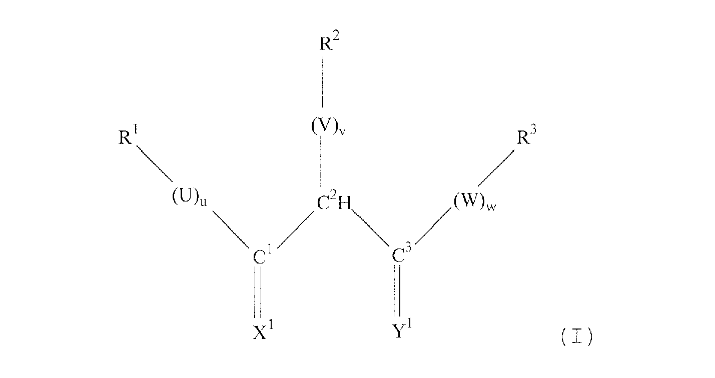 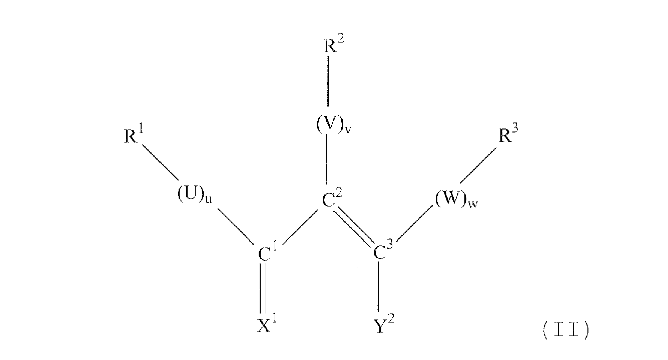 in which:
X¹ and Y¹ represent, independently of one another, an oxygen or sulphur atom or an =NH group;
Y² represents an -OH, -SH or -NH₂ group; u, v and w are, independently of one another, 0 or 1, with the proviso, however, that at least one of u, v and w is other than 0;
U, V and W represent, independently of one another, an aryl or heteroaryl group having one or more rings, each having 5 or 6 ring members;
at least one of R¹, R² and R³ represents a group -(Z)_{z}K in which:
* z is 0 or 1, Z represents a C₃ to C₆ cyclic, or C₁ to C₆ linear or branched, saturated or unsaturated divalent hydrocarbon group, this hydrocarbon group optionally containing one or more oxygen, nitrogen or sulphur atoms; and
* K represents:
• either a group -Si(Cl)ₘ(OR⁴)ₙ(R⁵)ₚ in which:
- m and n are integers ranging from 0 to 3, p is an integer ranging from 0 to 2, with the proviso, however, that m + n is equal to 1, 2 or 3 and m + n + p is equal to 3;
- R⁴ and R⁵ represent, independently of one another, a C₃ to C₆ cyclic, or C₁ to C₆ linear or branched, saturated or unsaturated hydrocarbon group, this hydrocarbon group optionally containing one or more oxygen, nitrogen or sulphur atoms, or else an aryl or heteroaryl group having a 5- or 6-membered ring;
• or a metal-complexing group;
and in which that or those of R¹, R² and R³ which does not or do not represent a group -(Z)_{z}K, if there is one, represent (s) a hydrogen or halogen atom, a -CN or -NO₂ group, a C₃ to C₆ cyclic, or C₁ to C₆ linear or branched, saturated or unsaturated hydrocarbon group, this hydrocarbon group optionally containing one or more oxygen, nitrogen or sulphur atoms, or else an aryl or heteroaryl group having a 5- or 6-membered ring; as probe molecule for the detection or quantitative determination of halogenated gaseous compounds.

26. Use according to Claim 25, for the detection or quantitative determination of halogenated boron complexes, in particular BF₃ and BCl₃.

## Patentansprüche

1. Verbindung gemäß wenigstens einer der nachstehenden allgemeinen Formeln (I) und (II): 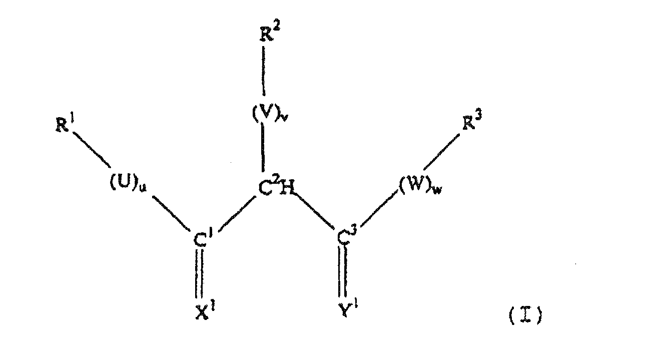 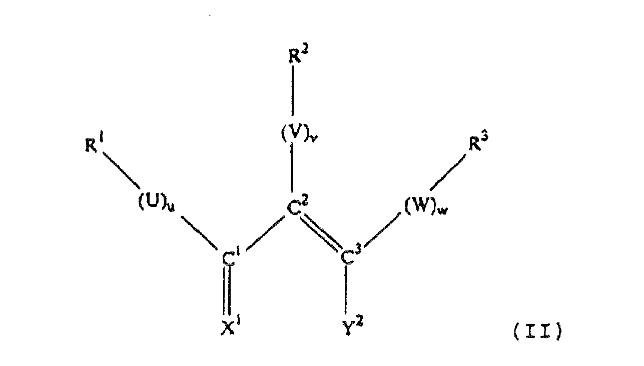 worin
X¹ und Y¹ unabhängig von einander ein Sauerstoff- oder Schwefelatom oder eine Gruppe =NH darstellen;
Y² eine Gruppe -OH, -SH oder -NH₂ darstellt;
u, v und w unabhängig von einander 0 oder 1 sind, jedoch mit der Maßgabe, daß wenigstens eines von u, v und w von 0 verschieden ist;
U, V und W unabhängig von einander eine Aryl- oder Heteroarylgruppe mit einem oder mehr Ringen mit jeweils 5 oder 6 Gliedern darstellen;
wenigstens eines von R¹, R² und R³ eine Gruppe -(Z)_{z}K darstellt, worin
* z 0 oder 1 ist, Z eine zweiwertige, gesättigte oder ungesättigte, gerade oder verzweigte C₁-C₆- oder cyclische C₃-C₆-Kohlenwasserstoffgruppe darstellt, wobei diese Kohlenwasserstoffgruppe gegebenenfalls ein oder mehr Sauerstoff-, Stickstoff- oder Schwefelatome umfaßt, und
* K
• entweder eine Gruppe -Si(Cl)ₘ(OR⁴)ₙ(R⁵)ₚ, worin
- m und n ganze Zahlen von 0 bis 3 sind, p eine ganze Zahl von 0 bis 2 ist, jedoch mit der Maßgabe, daß m + n gleich 1, 2 oder 3 ist und m + n + p gleich 3 ist;
- R⁴ und R⁵ unabhängig voneinander eine gesättigte oder ungesättigte, gerade oder verzweigte C₁-C₆- oder cyclische C₃-C₆-Kohlenwasserstoffgruppe, wobei diese Kohlenwasserstoffgruppe gegebenenfalls ein oder mehr Sauerstoff-, Stickstoff- oder Schwefelatome umfaßt, oder eine Aryl- oder Heteroarylgruppe mit einem Ring mit 5 oder 6 Gliedern darstellen;
• oder eine Metallkomplexierungsgruppe darstellt, und worin das oder die R¹, R² und R³, die die keine Gruppe -(Z)_{z}K darstellen, falls vorhanden, ein Wasserstoff- oder Halogenatom, eine Gruppe -CN oder -NO₂, eine gesättigte oder ungesättigte, gerade oder verzweigte C₁-C₆- oder cyclische C₃-C₆-Kohlenwasserstoffgruppe, wobei diese Kohlenwasserstoffgruppe gegebenenfalls ein oder mehr Sauerstoff-, Stickstoff- oder Schwefelatome umfaßt, oder eine Aryl- oder Heteroarylgruppe mit einem Ring mit 5 oder 6 Gliedern darstellen;
mit Ausnahme
- einer Verbindung der allgemeinen Formel (I), worin R³ eine Gruppe -O(CH₂)₃-Si(OCH₂CH₃)₂CH₃ darstellt, wenn X¹ und Y¹ alle beide ein Sauerstoffatom darstellen, u und w alle beide 1 sind, U und W alle beide eine Phenylgruppe darstellen, v 0 ist, und R¹ und R² alle beide ein Wasserstoffatom darstellen, und
- einer Verbindung der allgemeinen Formel (I), worin R³ eine Gruppe -O(CH₂)₃-Si(OCH₂CH₃)₃ oder -O(CH₂)₃-Si(OCH₂CH₃)₂CH₃ darstellt, wenn X¹ und Y¹ alle beide ein Sauerstoffatom darstellen, u und w 1 sind, U und W alle beide eine Phenylgruppe darstellen, v 0 ist, R¹ eine tert-Butylgruppe darstellt und R² ein Wasserstoffatom darstellt.

2. Verbindung gemäß Anspruch 1, die wenigstens einer der allgemeinen Formeln (I) und (II) entspricht, worin X¹ und Y¹ ein Sauerstoffatom darstellen, Y² eine Gruppe -OH darstellt und u, v, w, U, V, W, R¹, R² und R³ wie vorstehend definiert sind.

3. Verbindung gemäß Anspruch 1 oder Anspruch 2, die wenigstens einer der allgemeinen Formeln (I) und (II) entspricht, worin v 0 ist, u und w 1 sind und U, W, X¹, Y¹, Y², R¹, R² und R³ wie vorstehend definiert sind.

4. Verbindung gemäß Anspruch 1 oder Anspruch 2, die wenigstens einer der allgemeinen Formeln (I) und (II) entspricht, worin u und v 0 sind, w 1 ist und W, X¹, Y¹, Y², R¹, R² und R³ wie vorstehend definiert sind.

5. Verbindung gemäß einem der Ansprüche 1 bis 4, wobei K der Gruppe -(Z)_{z}K, die wenigstens eines von R¹, R² und R³ bildet, eine aus den Gruppen -SiCl₃, -SiCl₂(OR⁴), -SiCl(OR⁴)₂ und -Si(OR⁴)₃ ausgewählte Gruppe darstellt, worin R⁴ eine C₁-C₆-, vorzugsweise C₁-C₃- und noch besser C₂-Alkylgruppe ist.

6. Verbindung gemäß einem der Ansprüche 1 bis 4, wobei K der Gruppe -(Z)_{z}K, die wenigstens eines von R¹, R² und R³ bildet, eine gesättigte oder ungesättigte, gerade oder verzweigte C₁-C₆- oder cyclische C₃-C₆-Kohlenwasserstoffgruppe darstellt, die durch mehrere, aus -OH, -COOH, -NH₂, =NOH, -SH, -PO₃H₂, -PO₂H, =O, =S, =N-, -NH- und -NH₂ ausgewählte funktionelle Gruppen substituiert ist.

7. Verbindung gemäß einem der Ansprüche 1 bis 6, die wenigstes einer der allgemeinen Formeln (1) und (11) entspricht, worin
X¹ und Y¹ alle beide ein Sauerstoffatom darstellen, Y² eine Gruppe -OH darstellt;
v 0 ist, u und w 1 sind;
U und W eine Phenylgruppe darstellen;
R¹ ein Wasserstoff- oder Halogenatom, eine Gruppe -CN oder -NO₂, eine gesättigte oder ungesättigte, gerade oder verzweigte C₁-C₆- oder cyclische C₃-C₆-Kohlenwasserstoffgruppe, wobei diese Kohlenwasserstoffgruppe gegebenenfalls ein oder mehr Sauerstoff-, Stickstoff- oder Schwefelatome umfaßt, oder eine Aryl- oder Heteroarylgruppe mit einem Ring mit 5 oder 6 Gliedern darstellt und R² ein Wasserstoffatom darstellt, während R³ eine Gruppe -(Z)_{z}K darstellt, worin z 1 ist, Z eine zweiwertige, gerade, gesättigte oder ungesättigte C₁-C₃-Kohlenwasserstoffgruppe darstellt und K wie vorstehend definiert ist.

8. Verbindung gemäß Anspruch 7, die 1-Phenyl-3-[4-(2-triethoxysifylvinyl)phenyl]-propan-1,3-dion ist.

9. Verbindung gemäß einem der Ansprüche 1 bis 6, die wenigstes einer der allgemeinen Formeln (I) und (II) entspricht, worin
X¹ und Y¹ alle beide ein Sauerstoffatom darstellen, Y² eine Gruppe -OH darstellt;
u und v 0 sind, w 1 ist;
W eine Phenylgruppe darstellt;
R¹ ein Wasserstoff- oder Halogenatom, eine Gruppe -CN oder -NO₂, eine gesättigte oder ungesättigte, gerade oder verzweigte C₁-C₆- oder cyclische C₃-C₆-Kohlenwasserstoffgruppe, wobei diese Kohlenwasserstoffgruppe gegebenenfalls ein oder mehr Sauerstoff-, Stickstoff- oder Schwefelatome umfaßt, oder eine Aryl- oder Heteroarylgruppe mit einem Ring mit 5 oder 6 Gliedern darstellt und R² ein Wasserstoffatom darstellt, während R³ eine Gruppe -(Z)_{z}K darstellt, worin z 1 ist, Z eine zweiwertige, gerade, gesättigte oder ungesättigte C₁-C₃-Kohlenwasserstoffgruppe darstellt und K wie vorstehend definiert ist.

10. Verbindung gemäß Anspruch 9, die 1-[4-(2-Triethoxysilylvinyl)phenyl]butan-1,3-dion ist.

11. Mesostrukturiertes, poröses organisch-anorganisches Hybridmaterial, das durch ein Verfahren des Sol-Gel-Typs erhalten werden kann, umfassend:
a) die Hydrolyse wenigstens einer Metallverbindung der nachstehenden allgemeinen Formel (III):
M(Cl)_{q}(OR⁶)ᵣ (III)
worin
M ein Metall ist;
q und r ganze Zahlen von 0 bis zur Wertigkeit von M sind, jedoch mit der Maßgabe, dass q + r gleich dieser Wertigkeit ist, und
R⁶ eine organische Gruppe darstellt,
in Lösung in einem Gemisch aus Wasser, einem organischen Lösungsmittel und einer Säure;
b) die Kondensation des in Schritt a) erhaltenen Produkts in Lösung in einem Gemisch, das außer Wasser, dem besagten organischen Lösungsmittel und der besagten Säure ein Tensid enthält;
c) die Reaktion des in Schritt b) erhaltenen Produkts mit wenigstens einer Verbindung gemäß wenigstens einer nachstehenden allgemeinen Formel (I) und (II): 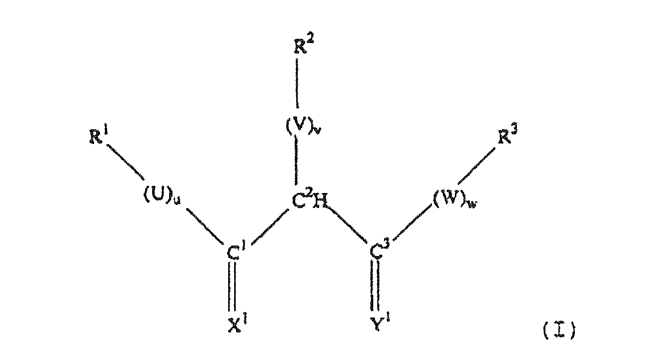 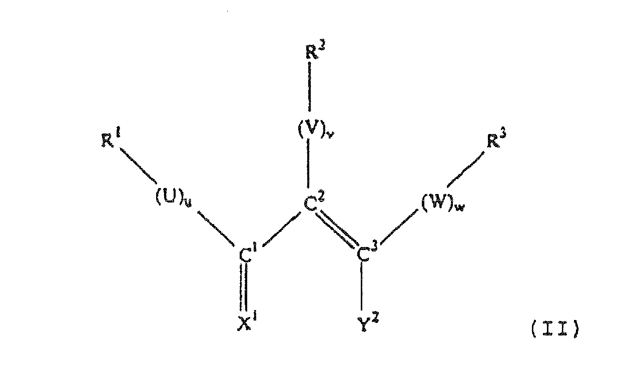 worin
X¹ und Y¹ unabhängig von einander ein Sauerstoff- oder Schwefelatom oder eine Gruppe =NH darstellen;
Y² eine Gruppe -OH, -SH oder -NH₂ darstellt;
u, v und w unabhängig von einander 0 oder 1 sind, jedoch mit der Maßgabe, daß wenigstens eines von u, v und w von 0 verschieden ist;
U, V und W unabhängig von einander eine Aryl- oder Heteroarylgruppe mit einem oder mehr Ringen mit jeweils 5 oder 6 Gliedern darstellen;
wenigstens eines von R¹, R² und R³ eine Gruppe -(Z)_{z}K darstellt, worin z 0 oder 1 ist, Z eine zweiwertige, gesättigte oder ungesättigte, gerade oder verzweigte C₁-C₆- oder cyclische C₃-C₆-Kohlenwasserstoffgruppe darstellt, wobei diese Kohlenwasserstoffgruppe gegebenenfalls ein oder mehr Sauerstoff-, Stickstoff- oder Schwefelatome umfaßt, und worin
* wenn M bei der in Schritt a) verwendeten Verbindung der allgemeinen Formel (III) Silizium oder Aluminium ist, K eine Gruppe -Si(Cl)ₘ(OR⁴)ₙ(R⁵)ₚ darstellt, worin m und n ganze Zahlen von 0 bis 3 sind, p eine ganze Zahl von 0 bis 2 ist, jedoch mit der Maßgabe, daß m + n gleich 1, 2 oder 3 ist und m + n + p gleich 3 ist; R⁴ und R⁵ unabhängig voneinander eine gesättigte oder ungesättigte, gerade oder verzweigte C₁-C₆- oder cyclische C₃-C₆-Kohlenwasserstoffgruppe, wobei diese Kohlenwasserstoffgruppe gegebenenfalls ein oder mehr Sauerstoff-, Stickstoff- oder Schwefelatome umfaßt, oder eine Aryl- oder Heteroarylgruppe mit einem Ring mit 5 oder 6 Gliedern darstellen; wohingegen
* wenn M bei der in Schritt a) verwendeten Verbindung der allgemeinen Formel (III) ein anderes Metall als Silizium ist, K eine Metallkomplexierungsgruppe darstellt, und worin das oder die R¹, R² und R³, die keine Gruppe -(Z)_{z}K darstellen, falls vorhanden, ein Wasserstoff- oder Halogenatom, eine Gruppe -CN oder -NO₂, eine gesättigte oder ungesättigte, gerade oder verzweigte C₁-C₆- oder cyclische C₃-C₆-Kohlenwasserstoffgruppe, wobei diese Kohlenwasserstoffgruppe gegebenenfalls ein oder mehr Sauerstoff-, Stickstoff- oder Schwefelatome umfaßt, oder eine Aryl- oder Heteroarylgruppe mit einem Ring mit 5 oder 6 Gliedern darstellen; und
d) eine Wärmebehandlung des Schritt c) erhaltenen Produkts,
wobei die Schritte a), b) und c) gleichzeitig ausgeführt werden können.

12. Mesostrukturiertes, poröses organisch-anorganisches Hybridmaterial gemäß Anspruch 11, das durch ein Verfahren erhalten werden kann, bei dem das Metall M der Verbindung der allgemeinen Formel (III) aus Silizium, Aluminium, Zirkonium, Titan, Niobium, Yttrium, Vanadium und Cerium ausgewählt ist.

13. Mesostrukturiertes, poröses organisch-anorganisches Hybridmaterial gemäß Anspruch 11 oder Anspruch 12, das durch ein Verfahren erhalten werden kann, bei dem die organische Gruppe R⁶ der Verbindung der allgemeinen Formel (111) eine C₁-C₆-, vorzugsweise C₁-C₃- und noch besser C₂-Alkylgruppe ist.

14. Mesostrukturiertes, poröses organisch-anorganisches Hybridmaterial gemäß einem der Ansprüche 11 bis 13, das durch ein Verfahren erhalten werden kann, bei dem das Metall M der Verbindung der allgemeinen Formel (111) Silizium ist.

15. Mesostrukturiertes, poröses organisch-anorganisches Hybridmaterial gemäß Anspruch 14, das durch ein Verfahren erhalten werden kann, bei dem K der Gruppe -(Z)_{z}K, die wenigstens eines von R¹, R² und R³ der wenigstens einer der allgemeinen Formeln (I) und (II) entsprechenden Verbindung bildet, eine aus den Gruppen -SiCl₃, -SiCl₂(OR⁴), -SiCl(OR⁴)₂ und -Si(OR⁴)₃ ausgewählte Gruppe darstellt, worin R⁴ eine C₁-C₆-, vorzugsweise C₁-C₃- und noch besser C₂-Alkylgruppe ist.

16. Mesostrukturiertes, poröses organisch-anorganisches Hybridmaterial gemäß einem der Ansprüche 11 bis 15, das durch ein Verfahren erhalten werden kann, bei dem Schritt a) unter Verwenden eines Alkohols, vorzugsweise Ethanol als organisches Lösungsmittel und Salzsäure als Säure und unter Erhitzen der Lösung der Verbindung der allgemeinen Formel (III) ausgeführt wird.

17. Mesostrukturiertes, poröses organisch-anorganisches Hybridmaterial gemäß einem der Ansprüche 11 bis 16, das durch ein Verfahren erhalten werden kann, bei dem Schritt b) bei Raumtemperatur unter Verwenden von Cetyltrimethylammoniumbromid als Tensid ausgeführt wird.

18. Mesostrukturiertes, poröses organisch-anorganisches Hybridmaterial gemäß einem der Ansprüche 11 bis 17, das durch ein Verfahren erhalten werden kann, bei dem Schritt c) durch Reagierenlassen der wenigstens einer der allgemeinen Formeln (I) und (II) entsprechenden Verbindung in Lösung in einem organischen Lösungsmittel mit dem in Schritt b) erhaltenen Produkt bei Raumtemperatur ausgeführt wird.

19. Mesostrukturiertes, poröses organisch-anorganisches Hybridmaterial gemäß einem der Ansprüche 11 bis 18, das durch ein Verfahren erhalten werden kann, bei dem die Wärmebehandlung in Schritt d) aus dem Aussetzen des sich aus Schritt c) ergebenden Produkts einer Temperatur von 120 bis 160 °C über eine Dauer von 24 bis 72 Stunden besteht.

20. Mesostrukturiertes, poröses organisch-anorganisches Hybridmaterial gemäß einem der Ansprüche 11 bis 19, das durch ein Verfahren erhalten werden kann, das außerdem zwischen dem Schritt c) und d) einen Schritt umfaßt, bei dem das in Schritt c) erhaltene Produkt in Form eines dünnen Films gebracht wird.

21. Mesostrukturiertes, poröses organisch-anorganisches Hybridmaterial gemäß einem der Ansprüche 11 bis 20, das durch ein Verfahren erhalten werden kann, bei dem Schritt a) die Hydrolyse mehrerer Verbindungen der allgemeinen Formel (111) M¹(Cl)_{q1}(OR⁶)ᵣ₁, M²(Cl)_{q2}(OR⁷)ᵣ₂, ..., Mⁿ(Cl)_{qn}(ORⁿ)ᵣₙ umfaßt, worin
M¹, M², ..., Mⁿ von einander verschiedene Metalle darstellen,
q1 und r1 ganze Zahlen von 0 bis zur Wertigkeit von M¹ mit q1 + r1 gleich dieser Wertigkeit sind,
q2 und r2 ganze Zahlen von 0 bis zur Wertigkeit von M² mit q2 + r2 gleich dieser Wertigkeit sind,
qn und m ganze Zahlen von 0 bis zur Wertigkeit von Mⁿ mit qn + rn gleich dieser Wertigkeit sind, und
R⁶, R⁷, ... , Rⁿ unabhängig voneinander eine organische Gruppe darstellen.

22. Verfahren zur Herstellung eines wie in einem der Ansprüche 11 bis 21 definierten mesostrukturierten, porösen organisch-anorganischen Hybridmaterials, das als empfindliches Material in einem Sensor zum Nachweis oder der Bestimmung gasförmiger, halogenierter Verbindungen geeignet ist.

23. Sonde, die zum Nachweis oder der Bestimmung gasförmiger, halogenierter Verbindungen geeignet ist und als empfindliches Material ein wie in einem der Ansprüche 11 bis 21 definiertes mesostrukturiertes, poröses organisch-anorganisches Hybridmaterial umfaßt.

24. Sonde gemäß Anspruch 23 zum Nachweis oder zur Bestimmung halogenierter Borkomplexe, insbesondere BF₃ und BCl₃.

25. Verwendung einer Verbindung, die wenigstes einer der nachstehenden allgemeinen Formeln (I) und (II) entspricht: 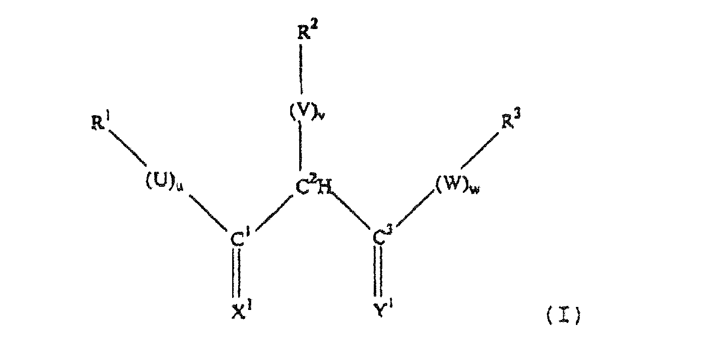 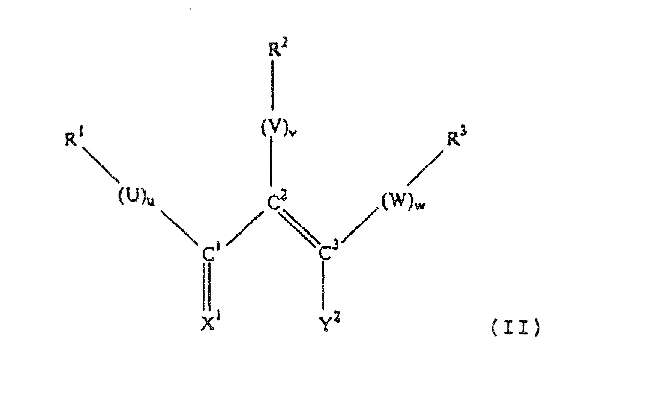 worin
X¹ und Y¹ unabhängig von einander ein Sauerstoff- oder Schwefelatom oder eine Gruppe =NH darstellen;
Y² eine Gruppe -OH, -SH oder -NH₂ darstellt;
u, v und w von einander unabhängig 0 oder 1 sind, jedoch mit der Maßgabe, daß wenigstens eines von u, v und w von 0 verschieden ist;
U, V und W unabhängig von einander eine Aryl- oder Heteroarylgruppe mit einem oder mehr Ringen mit jeweils 5 oder 6 Gliedern darstellen;
wenigstens eines von R¹, R² und R³ eine Gruppe -(Z)_{z}K darstellt, worin
* z 0 oder 1 ist, Z eine zweiwertige, gesättigte oder ungesättigte, gerade oder verzweigte C₁-C₆- oder cyclische C₃-C₆-Kohlenwasserstoffgruppe darstellt, wobei diese Kohlenwasserstoffgruppe gegebenenfalls ein oder mehr Sauerstoff-, Stickstoff- oder Schwefelatome umfaßt, und
* K
• entweder eine Gruppe -Si(Cl)ₘ(OR⁴)ₙ(R⁵)ₚ, worin
- m und n ganze Zahlen von 0 bis 3 sind, p eine ganze Zahl von 0 bis 2 ist, jedoch mit der Maßgabe, daß m + n gleich 1, 2 oder 3 ist und m + n + p gleich 3 ist;
- R⁴ und R⁵ unabhängig voneinander eine gesättigte oder ungesättigte, gerade oder verzweigte C₁-C₆- oder cyclische C₃-C₆-Kohlenwasserstoffgruppe, wobei diese Kohlenwasserstoffgruppe gegebenenfalls ein oder mehr Sauerstoff-, Stickstoff- oder Schwefelatome umfaßt, oder eine Aryl- oder Heteroarylgruppe mit einem Ring mit 5 oder 6 Gliedern darstellen;
• oder eine Metallkomplexierungsgruppe darstellt, und worin das oder die R¹, R² und R³, die die keine Gruppe -(Z)_{z}K darstellen, falls vorhanden, ein Wasserstoff- oder Halogenatom, eine Gruppe -CN oder -NO₂, eine gesättigte oder ungesättigte, gerade oder verzweigte C₁-C₆- oder cyclische C₃-C₆-Kohlenwasserstoffgruppe, wobei diese Kohlenwasserstoffgruppe gegebenenfalls ein oder mehr Sauerstoff-, Stickstoff- oder Schwefelatome umfaßt, oder eine Aryl- oder Heteroarylgruppe mit einem Ring mit 5 oder 6 Gliedern darstellen,
als Sondenmolekül zum Nachweis oder der Bestimmung gasförmiger, halogenierter Verbindungen.

26. Verwendung gemäß Anspruch 25 zum Nachweis oder der Dosierung halogenierter Borkomplexe, insbesondere BF₃ und BCl₃.
